(19) **Europäisches Patentamt / European Patent Office / Office européen des brevets**

(11) **EP 2 528 499 B1**

(12) **EUROPEAN PATENT SPECIFICATION**

(45) Date of publication and mention
of the grant of the patent:
**01.01.2020 Bulletin 2020/01**

(21) Application number: **11737698.8**

(22) Date of filing: **28.01.2011**

(51) Int Cl.:
*A61B 5/0205* (2006.01)      *A61B 5/0452* (2006.01)
*A61B 5/08* (2006.01)

(86) International application number:
**PCT/US2011/022850**

(87) International publication number:
**WO 2011/094487 (04.08.2011 Gazette 2011/31)**

(54) **ELIMINATION OF THE EFFECTS OF IRREGULAR CARDIAC CYCLES IN THE DETERMINATION OF CARDIOVASCULAR PARAMETERS**

ELIMINIERUNG DER EFFEKTE IRREGULÄRER HERZZYKLEN BEI DER BESTIMMUNG VON HERZ-KREISLAUF-PARAMETERN

ELIMINATION DES EFFETS DE CYCLES CARDIAQUES IRRÉGULIERS LORS DE LA DÉTERMINATION DE PARAMÈTRES CARDIOVASCULAIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.01.2010 US 299428 P**

(43) Date of publication of application:
**05.12.2012 Bulletin 2012/49**

(73) Proprietor: **Edwards Lifesciences Corporation
Irvine, CA 92614 (US)**

(72) Inventors:
• **JIAN, Zhongping
Irvine, CA 92614 (US)**

• **HATIB, Feras
Irvine, CA 92614 (US)**

(74) Representative: **Eisenführ Speiser
Patentanwälte Rechtsanwälte PartGmbB
Postfach 31 02 60
80102 München (DE)**

(56) References cited:
**US-A1- 2003 109 791      US-A1- 2003 187 479
US-A1- 2004 193 066      US-A1- 2005 187 481
US-A1- 2008 167 567      US-A1- 2009 048 527
US-A1- 2009 048 527**

## Description

## BACKGROUND

Indicators such as stroke volume (SV), cardiac output (CO), end-diastolic volume, ejection fraction, stroke volume variation (SVV), pulse pressure variation (PPV), and systolic pressure variations (SPV), among others, are important not only for diagnosis of disease, but also for "real-time" monitoring of preload dependence, fluid responsiveness, or volume responsiveness condition of both human and animal subjects. Few hospitals are therefore without some form of equipment to monitor one or more of these cardiac parameters. Many techniques, including invasive techniques, non-invasive techniques, and combinations thereof, are in use and even more have been proposed in the literature.

Many of the techniques used to measure SV can be adapted to provide an estimate of CO as well, because CO is generally defined as SV times the heart rate (HR), which is usually available to monitoring equipment. Conversely, most devices that estimate CO also estimate SV in their calculations. One way to estimate SVV is simply to collect multiple SV values and calculate the differences from measurement interval to measurement interval.

One way to measure SV or CO is to mount a flow-measuring device on a catheter, and position the device in or near the subject's heart. Some such devices inject either a bolus of material or energy (usually heat) at an upstream position, such as in the right atrium, and determine flow based on the characteristics of the injected material or energy at a downstream position, such as in the pulmonary artery. Patents that disclose implementations of such invasive techniques (in particular, thermodilution) include: U.S. Pat. No. 4,236,527 (Newbower et al., 2 Dec. 1980); U.S. Pat. No. 4,507,974 (Yelderman, 2 Apr. 1985); U.S. Pat. No. 5,146,414 (McKown, et al., 8 Sep. 1992); and U.S. Pat. No. 5,687,733 (McKown, et al., 18 Nov. 1997). Other invasive devices are based on the known Fick technique, according to which CO is calculated as a function of oxygenation of arterial and mixed venous blood.

Invasive techniques have obvious disadvantages, especially when the subjects in need of such monitoring are already in the hospital due to a serious condition. Invasive methods also have less obvious disadvantages, for example, some techniques such as thermodilution rely on assumptions, such as uniform dispersion of the injected heat, which can affect the accuracy of the measurements. Moreover, the introduction of an instrument into the blood flow may affect the value that the instrument measures.

Doppler techniques, using invasive as well as non-invasive transducers, have also been used to obtain flow rate data that can then be used to calculate SV and CO. However, these systems are typically expensive, and their accuracy depends on precise knowledge of the diameter and general geometry of the flow channel. Such precise knowledge is, however, seldom possible, especially under conditions where real-time monitoring is desired.

One blood characteristic that can be obtained with minimal or no invasion is blood pressure. In addition to causing minimal patient trauma, blood pressure measurement technology has the added benefit of being accurate.

Many blood pressure measurement systems rely on the pulse contour method (PCM), which calculates an estimate of one or more cardiac parameters of interest, such as CO, from characteristics of a blood pressure waveform. In the PCM, "Windkessel" parameters, such as characteristic impedance of the aorta, compliance, and total peripheral resistance, are often used to construct a linear or non-linear hemodynamic model of the aorta. In essence, blood flow is analogized to a flow of electrical current in a circuit in which an impedance is in series with a parallel-connected resistance and capacitance (compliance). The three required parameters of the model are usually determined either empirically, through a complex calibration process, or from compiled "anthropometric" data, *i.e.,* data about the age, sex, height, weight, and/or other parameters of other patients or test subjects. U.S. Pat. No. 5,400,793 (Wesseling, 28 Mar. 1995) and U.S. Pat. No. 5,535,753 (Petrucelli,.et al., 16 Jul. 1996) disclose systems that rely on a Windkessel circuit model to determine CO.

PCM-based systems can monitor SV-derived cardiac parameters using blood pressure measurements taken using a variety of measurement apparatus, such as a finger cuff, and can do so more or less continuously. This ease of use comes at the potential cost of accuracy, however, as the PCM can be no more accurate than the rather simple, three-parameter model from which it was derived. A model of a much higher order would be needed to faithfully account for other phenomena. Many improvements, with varying degrees of complexity, have been proposed for improving the accuracy of the basic PCM model.

Recently, several studies have confirmed the clinical significance of monitoring the variations observed in left ventricular stroke volume that result from the interaction of the cardiovascular system and the lungs under mechanical ventilation. These stroke volume variations (SVV) are caused by the cyclic increases and decreases in the intrathoracic pressure due to the mechanical ventilation, which lead to variations in the cardiac preload and afterload. SVV has recently been extensively investigated and several studies have shown the usefulness of using SVV as a predictor of preload dependence and fluid responsiveness in various clinical situations. Several other parameters based on SVV have been found to be useful as well. In particular, systolic pressure variation (SPV) with its delta-Up ($\Delta$Up) and delta-Down ($\Delta$Down) components has been found to be a very useful predictor of preload dependence and fluid responsiveness. SPV is based on the changes in the arterial pulse pressure due to respiration-induced variations in stroke volume. Yet another parameter that has recently been investigated and shown to be a valid indicator of preload dependence and fluid responsiveness is the pulse pressure variation (PPV).

These recent developments in arterial pulse contour analysis methods have opened unique opportunities for less-invasive, continuous and real-time estimation of SVV. This allows clinicians to use SVV routinely along with SV and CO in their assessment of the hemodynamic state of critical care patients.

Existing systems for measuring preload dependence and fluid responsiveness based on respiration-induced changes in the arterial pulse pressure are almost all based on one of only a few methods. Some of the methods described in the literature include the measurements of Pulse Pressure Variation (PPV), Systolic Pressure Variation (SPV) and Stroke Volume Variation (SVV).

PPV estimation is based on Equation 1:

$$PPV = 100 \times \left\{ \frac{(PP_{max} - PP_{min})}{\left[ \frac{1}{2}(PP_{max} + PP_{min}) \right]} \right\} \qquad \text{(Equation 1)}$$

where PP is the measured pulse pressure, and $PP_{max}$ and $PP_{min}$ are, respectively, the maximum and the minimum values of the pulse pressure during one respiratory (inspiration-expiration) cycle. PP is calculated as the difference between the maximum and the minimum pressure during one cardiac cycle.

SPV estimation is based on Equation 2:

$$SPV = 100 \times \left\{ \frac{(SP_{max} - SP_{min})}{\left[ \frac{1}{2}(SP_{max} + SP_{min}) \right]} \right\} \qquad \text{(Equation 2)}$$

where SP is the measured systolic pressure, and $SP_{max}$ and $SP_{min}$ are, respectively, the maximum and minimum values of the systolic pressure during one respiratory cycle.

Similarly, SVV estimation is based on Equation 3:

$$SVV = 100 \times \left\{ \frac{(SV_{max} - SV_{min})}{\left[ \frac{1}{2}(SV_{max} + SV_{min}) \right]} \right\} \qquad \text{(Equation 3)}$$

where SV is the stroke volume, and $SV_{max}$ and $SV_{min}$ are, respectively, the maximum and minimum values of the stroke volume during one respiratory cycle.

In Equations 1, 2, and 3, the denominators are the averages of the maximum and minimum values of PP, SP and SV, respectively. In other words, the denominators are mean values, albeit of only two measurement points. This simple averaging of extreme values has been most common merely to simplify the calculations, which have typically been performed by hand. More reliable values may be obtained, however, by using the mean of all the measurement values over the measurement interval, that is, the first statistical moment of PP, SP, and SV.

Thus, for each of PPV, SPV and SVV, the respective variation value formula expresses the magnitude of the range of the value (maximum minus minimum) relative to the mean of the extreme (maximum and minimum) values.

U.S. Pat. No. 7,422,562 discloses further methods for calculating SVV including a method that uses the standard deviation of the arterial pressure cycles to measure SVV based on Equation 4:

$$SVV = 100 \times \left\{ \frac{(std(P)_{max} - std(P)_{min})}{std(P)_{mean}} \right\} \qquad \text{(Equation 4)}$$

where $std(P)_{max}$ and $std(P)_{min}$ are, respectively, the maximum and minimum standard deviation of the arterial pressure during one respiratory cycle and $std(P)_{mean}$ is the mean standard deviation of the arterial pressure signal in the same respiratory cycle. The standard deviation is computed as the standard deviation of all the pressure samples within a single cardiac cycle. U.S. Patent No. 7,422,562 also discloses an alternative method to measure SVV using the standard deviation of the arterial pressure signal based on equation 5:

$$SVV = 100 \times C \times \left\{ \frac{std(std(P))}{mean(std(P))} \right\}$$  (Equation 5)

where std(std(P)) is the standard deviation of the standard deviation of the arterial pressure beats during a period of time containing at least one respiratory cycle, mean(std(P)) is the mean of the standard deviation of the arterial pressure beats for the same period of time and C is an empirically derived constant.

The specific monitoring of SVV has both specific difficulties and advantages. Physiologically, SVV is based on several complex mechanisms of cardio-respiratory interaction. In brief: mechanical ventilation causes changes in left ventricular preload, which leads to distinct variations in left ventricular stroke cardio-respiratory interaction. In brief: mechanical ventilation causes changes in left ventricular preload, which leads to distinct variations in left ventricular stroke volume and systolic arterial pressure. Monitoring of SVV enables prediction of left ventricular response to volume administration and helps with correct assessment of hypovolemia and the subsequent decision to undertake volume resuscitation in many critical situations.

US 2003/109791 A1 relates to a biological data observation apparatus including a measuring unit including at least a photoelectric sensor including a light emitting element for emitting light with a predetermined wavelength onto a blood vessel of a subject and a light detecting element for detecting, as a photoelectric volume pulse wave, a change in an amount of transmitted or reflected light resulting from the light emitted from the light emitting section, a storage area in which pressure conversion data correlating a pulse wave area and a blood pressure value in order that the pulse wave area may be converted to the blood pressure value as an absolute value, the pulse wave area being obtained by integrating a wave form of the photoelectric capacity pulse wave per heartbeat,; an operational unit calculating the pulse wave area on the basis of the photoelectric volume pulse wave obtained from the subject and further calculating the blood pressure value of the subject on the basis of the calculated pulse wave area and the blood pressure conversion data, and a display unit displaying a result of calculation performed by the operational unit.

US 2005/0187481 A1 relates to a pre-processing arrangement for improving reliability of estimates of more general cardiac or hemodynamic parameters which involves smoothing with an approximating function, and sampling and low-pass filtering at an adjustable rate.

US 2009/048527 A1 relates to methods for determining a cardiovascular parameter reflecting fluid or volume changes and for detecting arrhythmia are disclosed. These methods involve receiving a waveform dataset corresponding to an arterial blood pressure, pulseox, Doppler ultrasound or bioimpedance signal and analyzing the waveform to detect premature ventrical or atrial contractions.

## SUMMARY

Methods for determining a cardiovascular parameter are disclosed. These methods involve first receiving a waveform dataset corresponding to an arterial blood pressure signal, or any signal proportional to, or derived from the arterial blood pressure signal, such as pulse oximetry (pulseox), Doppler ultrasound, or bioimpedance signal. The methods then involve identifying individual cardiac cycles in the waveform dataset, measuring the waveform characteristics for the individual cycles, and determining if the individual cardiac cycles are regular cardiac cycles or irregular cardiac cycles. Once the cardiac cycles are classified to regular and irregular cardiac cycles, a respiratory parameter is measured. Next, a modified waveform dataset containing the waveform characteristics of the regular cardiac cycles and the waveform characteristics of the irregular cardiac cycles is created wherein the waveform characteristics of the irregular cardiac cycles are replaced with estimated waveform characteristics. The estimated waveform characteristics used to create the modified waveform are calculated based on the waveform characteristics of the regular cardiac cycles. Finally, a cardiovascular parameter is calculated using the modified waveform dataset.

According to a first aspect of the present invention, there is disclosed a method of determining a cardiovascular parameter according to claim 1.

If the individual cardiac cycles are regular cardiac cycles or irregular cardiac cycles comprises the steps of comparing one or more waveform characteristics of the individual cardiac cycle to one or more waveform characteristics of a control cardiac cycle; and identifying the individual cardiac cycle as an irregular cardiac cycle if the one or more waveform characteristics of the individual cardiac cycle differs from the one or more waveform characteristics of the control cardiac cycle by a predetermined threshold amount.

In the present method, replacing the waveform characteristics of the irregular cardiac cycles with estimated waveform characteristics comprises the steps of determining pseudo-starting points and/or pseudo-ending points for the irregular cardiac cycles; and directly measuring or calculating waveform parameters/characteristics of the irregular cardiac cycle based on the pseudo-starting points and/or pseudo-ending points.

According to the present method, determining the pseudo-ending point for an irregular cycle comprises determining a time factor for all the regular cardiac cycles; determining a pressure factor based on the diastolic pressures of the cycles; identifying a starting point of the irregular cycle and pressure data points that are within a pre-established range from the starting point; if there is a pressure data point within the pre-established range and the relationship between the pressure data point and the pressure factor is within a predetermined range, that pressure data point is assigned to be the pseudo-ending point; and if there is more than one pressure data point within the range and the relationship between each of the more than one pressure data points and the pressure factor is within a predetermined range, the pressure data point with a time value that is closest to the time factor is assigned to be the pseudo-ending point.

The pre-established range from the starting point is the starting point plus (a range start factor * the median time duration) to the starting point plus (a range stop factor * the median time duration). The range start factor is 0.9 and the range stop factor is 1.1.

Moreover, in accordance with the present method, determining the pseudo-starting point for an irregular cycle comprises the steps of determining a time factor for all the regular cardiac cycles; determining a pressure factor based on the diastolic pressures of the cycles; identifying an ending point of the irregular cycle and pressure data points for the waveform that are within a pre-established range from the ending point; if there is a pressure data point within the pre-established range and the relationship between the pressure data point and the pressure factor is within a predetermined range, that pressure data point is assigned to be the pseudo-starting point; and if there is more than one pressure data point within the range and the relationship between each of the more than one pressure data points and the pressure factor is within a predetermined range, the pressure data point with a time value that is closest to the time factor is assigned to be the pseudo-ending point.

The pre-established range from the starting point is the starting point plus (a range begin factor * the median time duration) to the starting point plus (a range end factor * the median time duration). The range begin factor is 1.1 and the range end factor is 0.9.

The waveform characteristicis the standard deviation of an irregular cycle.

According to the present method, the standard deviation of an irregular cycle is calculated by determining the systolic pressure of the nearest regular cycle; determining the standard deviation of the nearest regular cycle; determining the systolic pressure of the irregular cycle from the respiratory parameter; and solving Equation 11.

The predetermined threshold amount is selected from one of 30% or more, 25% or more, 20% or more, 15% or more, 10% or more, 5% or more and 1% or more.

Further according to the present method the irregular cardiac cycle is a premature ventricular contraction, a premature atrial contraction, a cardiac cycle caused by arrhythmia, a cardiac cycle caused by atrial fibrillation, a patient artifact or a missing cardiac cycle.

The patient artifact is related to patient movement, electrical interference, or signal noise.

In a further aspect of the present invention, the control cardiac cycle is a regular cardiac cycle immediately preceding the individual cardiac cycle. In this case, the method comprises the step of comparing the individual cardiac cycle to a regular cardiac cycle immediately following the individual cardiac cycle.

The control cardiac cycle may be a regular cardiac cycle immediately following the individual cardiac cycle, a median cardiac cycle from a sequence containing at least three cardiac cycles or a mean cardiac cycle from a sequence containing at least three cardiac cycles.

According to the present method, the one or more waveform characteristics is a statistical measurement of a phase of a cardiac cycle. The statistical measurement is one of average, variance, skewness, or kurtosis. The phase of a cardiac cycle is one of the entire cardiac cycle, systole, diastole, systolic rise, systolic decay, or overall decay.

The one or more waveform characteristics is a time interval of the phase of a cardiac cycle. The time interval is measured from the time corresponding to the end of the diastolic phase of the previous cardiac cycle.

According to the method of the present invention, the one or more waveform characteristics is the power of a phase of a cardiac cycle. The phase of a cardiac cycle is selected from the group consisting of the entire cardiac cycle, systole, diastole, systolic rise, systolic decay, and overall decay.

Further according to the method of the present invention, the one or more waveform characteristics is one or more frequency characteristics of a phase of a cardiac cycle. The phase of a cardiac cycle is selected from the group consisting of the entire cardiac cycle, systole, diastole, systolic rise, systolic decay, and overall decay.

Moreover, the one or more waveform characteristics is one or more time-frequency characteristics of a phase of a cardiac cycle. The phase of a cardiac cycle is selected from the group consisting of the entire cardiac cycle, systole, diastole, systolic rise, systolic decay, and overall decay.

The one or more waveform characteristics may be a value corresponding to the maximum pressure of a cardiac cycle.

The the one or more waveform characteristics may include characteristics corresponding to the maximum pressure of a cardiac cycle and a time interval of the phase of a cardiac cycle. In this case, the phase of a cardiac cycle is selected from the group consisting of the entire cardiac cycle, systole, diastole, systolic rise, systolic decay, and overall decay.

In a further aspect of the present invention, the one or more waveform characteristics is the difference between a value corresponding to the diastolic pressure of the beginning of a cardiac cycle and a value corresponding to a maximum pressure of the cardiac cycle.

The one or more waveform characteristics may also be the difference between a value corresponding to the maximum pressure of a cardiac cycle and a value corresponding to the diastolic pressure at the end of a cardiac cycle.

In the method according to the present invention, the cardiovascular parameter is stroke volume variation, pulse pressure variation, or systolic pressure variation.

The cardiovascular parameter may be one of stroke volume, cardiac output, systemic vascular compliance, cardiac flow, cardiac flow velocity, vascular compliance or vascular elastance.

The method according to the present invention further comprises the step of filtering the waveform dataset with a low-pass filter.

The method further comprises indicating the position of the estimated cardiac cycles on a graphical user interface.

When an irregular cardiac cycle is detected, the inventive method further comprises the step of indicating that the waveform contains estimated cardiac cycles on a graphical user interface.

According to the inventive method, the waveform dataset is from a sampling period of a set duration. When an irregular cardiac cycle is detected, the duration of the sampling period is increased.

In one aspect of the inventive method, the signal proportional to, or derived from, the arterial blood pressure signal may be a pulseox, Doppler ultrasound, or bioimpedance signal.

In a further aspect, the respiratory parameter is the respiratory-induced variation in the waveform dataset caused by mechanical ventilation. The mechanical ventilation may be pressure controlled ventilation, volume controlled ventilation, synchronized intermittent mandatory ventilation, pressure support ventilation, volume support ventilation, high frequency ventilation, synchronized intermittent positive pressure ventilation, continuous positive pressure ventilation, or non-invasive ventilation.

In the method according to the present invention, the respiratory parameter is the respiratory-induced variation in the waveform dataset caused by spontaneous breathing.

Moreover, the estimated waveform characteristics are calculated using mathematical interpolation. The mathematical interpolation may be spline interpolation, polynomial interpolation, exponential interpolation, linear interpolation, nonlinear interpolation, piecewise interpolation, or multivariate interpolation.

Further according to the present invention, the estimated waveform characteristics may be calculated using mathematical curve fitting methods or by using approximating functions.

## DESCRIPTION OF DRAWINGS

Fig. 1 is an arterial pressure versus time (1/100$^{th}$ second increments) waveform displaying several cardiac cycles.

Fig. 2 is an arterial pressure versus time (1/100th second increments) waveform that contains two irregular cardiac cycles, in this case premature ventricular contractions.

Fig. 3 is an arterial pressure versus time (1/100th second increments) waveform showing two regular and one irregular cardiac cycles.

Fig. 4 is an arterial pressure versus time (1/100th second increments) waveform annotated to indicate the duration of an irregular cardiac cycle ($t_c$).

Fig. 5 is an arterial pressure versus time (1/100th second increments) waveform annotated to indicate the duration of a systole ($t_s$) and the duration of a diastole ($t_d$) of an irregular cardiac cycle.

Fig. 6 is an arterial pressure versus time (1/100th second increments) waveform annotated to indicate the duration of a systolic rise ($t_r$) and the duration of a systolic decay ($t_{dec}$) of an irregular cardiac cycle.

Fig. 7 is an arterial pressure versus time (1/100th second increments) waveform annotated to indicate the duration of the overall decay ($t_{ov\_dec}$) of an irregular cardiac cycle.

Fig. 8 is an arterial pressure versus time waveform with no irregular cardiac cycles also showing a calculated respiration parameter.

Fig. 9 is an arterial pressure versus time waveform with irregular cardiac cycles.

Fig. 10 is an arterial pressure versus time waveform with irregular cardiac cycles that are detectable based on pulse rate.

Fig. 11 is an arterial pressure versus time waveform with irregular cardiac cycles that are detectable based on systolic pressure.

Fig. 12 is an arterial pressure versus time waveform with one irregular cardiac cycle that is detectable based on pulse pressure.

Fig. 13 is an arterial pressure versus time waveform with irregular cardiac cycles that are detectable based on both systolic pressure and pulse rate.

Fig. 14 is an arterial pressure versus time waveform with irregular cardiac cycles that are detectable based on different left-side pulse pressure compared to right-side pulse pressure.

Fig. 15 is an arterial pressure versus time waveform with irregular cardiac cycles also showing a calculated respiration parameter.

Fig. 16 is a block diagram showing the main components of a system to implement the methods described herein.

Like reference numerals and symbols in the various drawings indicate like elements.

## DETAILED DESCRIPTION

As demonstrated by equations 1 through 5 above, the regularity, consistency and reliability of the cardiac cycles are importance for the accurate assessment of SVV, PPV, SPV, and other cardiovascular parameters. For example, the occurrence of irregular cardiac cycles in the measurement cycles, such as, for example, irregular cardiac cycles caused by cardiac arrhythmias, premature ventricular contractions, atrial fibrillation, or cardiac cycles affected by noise, electrical interference or motion artifacts, can significantly affect the accuracy of SVV, PPV, SPV, or other cardiovascular parameters as preload responsiveness indicators. For example, in cases of cardiac arrhythmia, the cardiac cycle-to-cardiac cycle variations in stroke volume reflect altered cardiac filling times rather than from the preload variation caused by mechanical ventilation. In those types of situations, SVV, PPV, SPV, or other cardiovascular parameters can not be used to predict preload dependence and fluid responsiveness. To overcome these problems, the methods disclosed herein eliminate the effect of irregular cardiac cycles and allows accurate measurement of preload dependence and fluid responsiveness in situations of high frequency occurrence of irregular cardiac cycles. Such accurate measurements were not possible with previously known techniques.
The methods disclosed herein determine a cardiovascular parameter, e.g., a parameter reflecting preload dependence and fluid responsiveness, using a modified waveform dataset. The waveform dataset corresponds to a signal, for example, from an arterial blood pressure, or any signal proportional to, or derived from the arterial pressure signal such as pulse-oximetry signal, Doppler ultrasound signal or bioimpedance signal. These methods involve identifying individual cardiac cycles in the waveform dataset, measuring the waveform characteristics for the individual cycles, then determining if the individual cardiac cycles are regular cardiac cycles or irregular cardiac cycles. Once the cardiac cycles are detected and classified to regular and irregular cardiac cycles, a respiratory parameter is measured. The respiratory parameter can be calculated using only the waveform characteristics of the regular cardiac cycles or can include waveform characteristics from the irregular cardiac cycles. Next, a modified waveform dataset containing the waveform characteristics of the regular cardiac cycles and the waveform characteristics of the irregular cardiac cycles is created wherein the waveform characteristics of the irregular cardiac cycles are replaced with estimated waveform characteristics. The estimated waveform characteristics used to create the modified waveform are calculated based on the waveform characteristics of the regular cardiac cycles and, as needed, the respiratory parameter. In some cases the estimated waveform characteristics are determined based only on the waveform characteristics of the regular cardiac cycles and in other cases the estimated waveform characteristics are determined based on both the waveform characteristics of the regular cardiac cycles and the respiratory parameter. Finally, a cardiovascular parameter is determined using the modified waveform dataset. Examples of irregular cardiac cycles that are detected and will have their waveform characteristics replaced in a waveform dataset by the methods described herein include, for example, premature ventricular contractions, premature atrial contractions, cardiac cycles caused by arrhythmia, cardiac cycles caused by atrial fibrillation, patient artifacts, noise from external interference, and missing cardiac cycles in those situations where the respective cardiac beats do not generate any flow or pressure. As used herein, the term waveform dataset refers to a set of data corresponding to a signal, *e.g.,* an arterial blood pressure signal, or any signal proportional to, or derived from the arterial blood pressure signal, such as pulse-oximetry (pulseox), Doppler ultrasound, or bioimpedance signal.

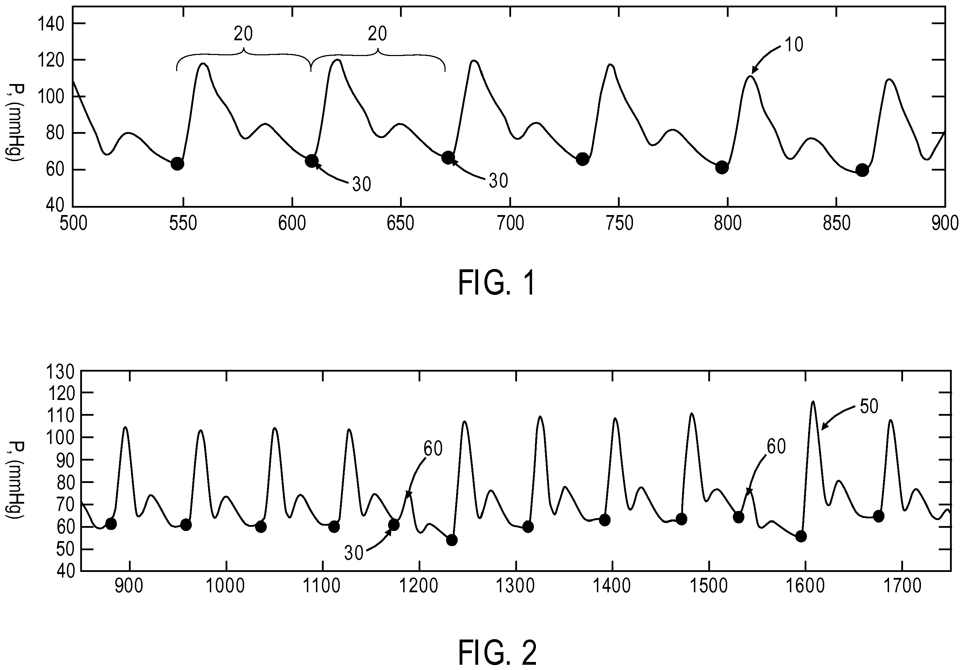

In the methods described herein, individual cardiac cycles (also called cardiac beats) are detected in the signal. Fig. 1 shows an example of an arterial pressure signal, in which the dots 30 along the arterial pressure signal 10 indicate the detected cardiac cycles which correspond to the end-diastolic pressure of the respective cardiac cycle and the starting point of the next cardiac cycle. The detection of the individual cardiac cycles in the signal can be based on real-time cardiac beat detection digital signal processing algorithms, such as those described in provisional patent application serial no. 61/151,670, filed February 11, 2009, which is incorporated herein in its entirety and the text of which is attached at Appendix A. These algorithms involve calculating the first derivative function of the signal and using a combination of an adaptive threshold and a zero-crossing detection to detect the beginning of the cardiac cycles.

The methods described herein then utilize a variety of methods for detecting irregular cardiac cycles. Detecting irregular cardiac cycles such as premature ventricular contractions, premature atrial contractions, cardiac cycles caused by arrhythmia, cardiac cycles caused by atrial fibrillation, patient artifacts, missing cardiac cycles, or noise from external interference, can be accomplished by identifying an individual cardiac cycle in a waveform dataset and comparing one or more waveform characteristics of the individual cardiac cycle to one or more waveform characteristics of a control cardiac cycle. Irregular cardiac cycles are identified by comparing the one or more parameters of an individual cardiac cycle with the same one or more parameters from a control cardiac cycle. If the one or more parameters of the individual cardiac cycle differ by a predetermined threshold amount from the same one or more parameters from the control cardiac cycle, the individual cardiac cycle is identified as an irregular cardiac cycle.

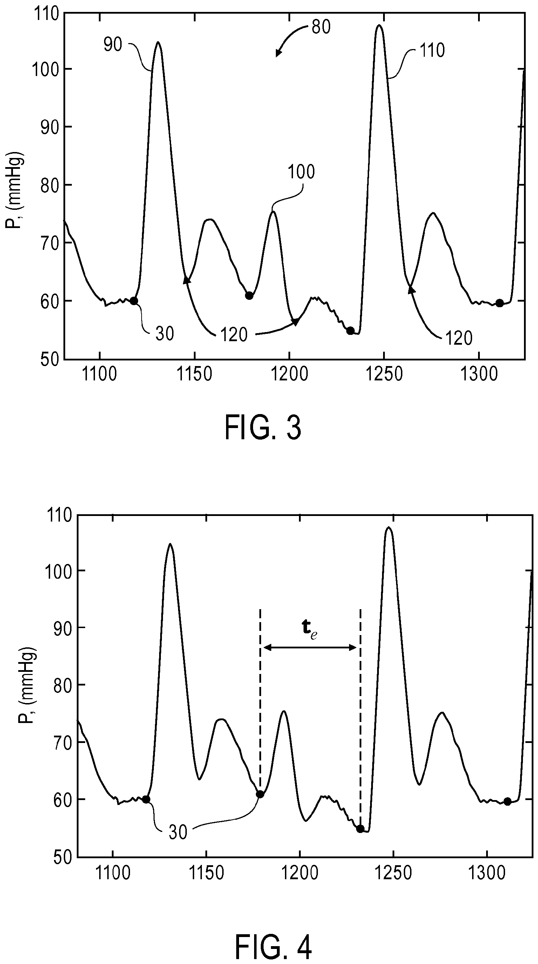

The parameters used for determining if individual cardiac cycles are regular cardiac cycles or irregular cardiac cycles are statistical and other measurements based on portions or phases of a cardiac cycle. Examples of phases of a cardiac cycle include the entire cardiac cycle, systole, diastole, systolic rise, systolic decay, or overall decay. The portions of a cardiac cycle used herein by way of example are shown in Figs. 1-7. In each of Figs. 1-7, the x-axis units are 100ths of a second (*e.g.,* 100 x-axis units corresponds to 1 second and 200 x-axis units corresponds to 2 seconds). Fig. 1 shows an arterial pressure waveform 10 with several cardiac cycles 20. The dots along the arterial pressure waveform 10 indicate the end-diastolic pressure 30 of one cardiac cycle and the starting point of the next cardiac cycle. Fig. 2 shows an arterial pressure waveform 50 with two irregular cardiac cycles corresponding to two premature ventricular contractions 60. The premature ventricular contractions 60 in Fig. 2 generate cardiac cycles with less pressure when compared to the other cardiac cycles. Fig. 3 shows an arterial pressure waveform 80 with three cardiac cycles (90, 100, and 110). The middle cardiac cycle 100 represents a premature ventricular contraction. The inflection point of an arterial pressure waveform of a cardiac cycle that defines the end of the systolic phase and the beginning of the diastolic phase is called a dichrotic notch 120.

The ending/starting point of a cardiac cycle 30 and the dichrotic notch 120 provide starting and ending points for defining various parameters used with the methods described herein, e.g., the time interval can be measured from the time corresponding to the end of the diastolic phase of the previous cardiac cycle to the end of the current diastolic phase. The parameters used herein include the entire cardiac cycle, and phases corresponding to the systole, the diastole, the systolic rise, the systolic decay, and the overall decay of an arterial pressure signal. The time components of each of these parameters are also used, *i.e.,* useful parameters include duration of the entire cardiac cycle ($t_c$), duration of the systole ($t_s$), duration of the diastole ($t_d$), duration of the systolic rise ($t_r$), duration of the systolic decay ($t_{dec}$), and duration of the overall decay ($t_{ov\_dec}$).

The duration of a cardiac cycle, $t_c$, is shown in Fig. 4. As shown, $t_c$ is the time between the starting point 30 of the cardiac cycle and the ending point of the cardiac cycle.

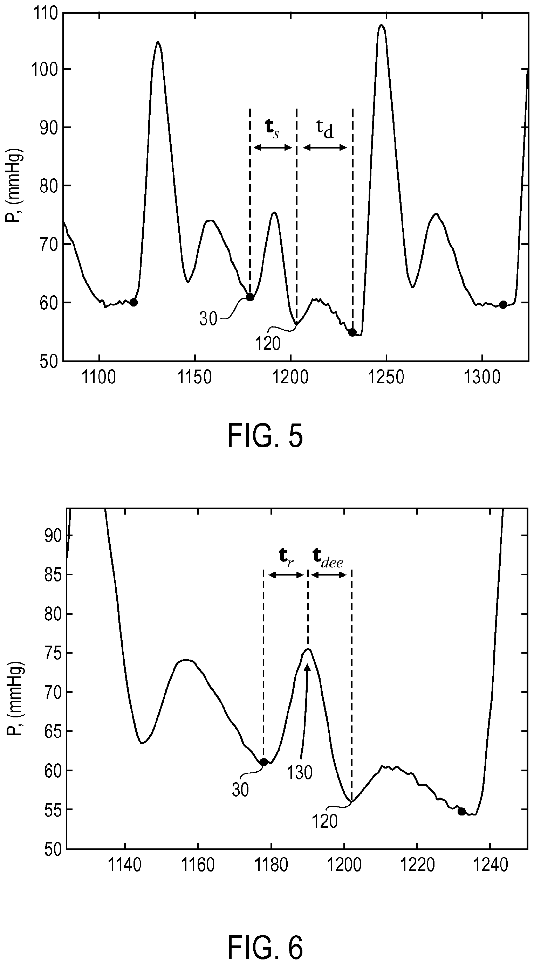

The duration of a systole, $t_s$, is shown in Fig. 5. As shown, $t_s$ is the time between the starting point 30 of the cardiac cycle and the dichrotic notch 120 of the cardiac cycle.

The duration of the diastole, $t_d$, is also shown in Fig. 5. As shown, $t_d$ is the time between the dichrotic notch 120 of the cardiac cycle and the ending point of the cardiac cycle.

The duration of a systolic rise, $t_r$, is shown in Fig. 6. As shown, $t_r$ is the time from the starting point 30 of the cardiac cycle to the maximum point 130 of the initial increase in arterial pressure after the onset of the systole.

The duration of the systolic decay, $t_{dec}$, is also shown in Fig. 6. As shown, $t_{dec}$ is the time from the maximum point 130 of the initial increase in arterial pressure after the onset of the systole to the dichrotic notch 120.

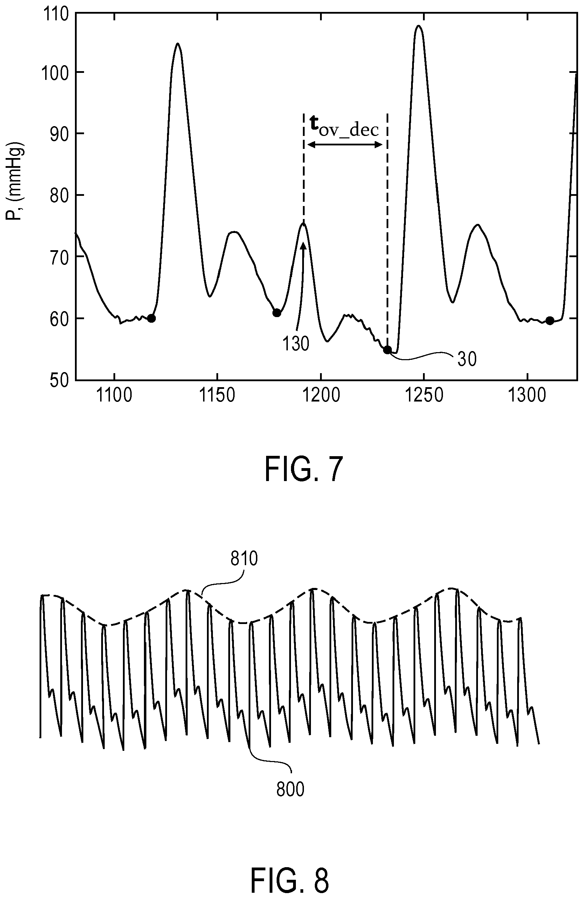

The duration of the overall decay, $t_{ov\_dec}$, is shown in Fig. 7. As shown, $t_{ov\_dec}$ is the time from the maximum point 130 of the initial increase in arterial pressure after the onset of the systole to the ending point of the cardiac cycle.

One method to detect an irregular cardiac cycle is to analyze the durations of the different phases of the cardiac cycle , *i.e.,* time intervals of the different phases, of an arterial waveform/signal as just described are compared. The methods described herein, for example, compare the duration of the entire cardiac cycle (i.e. the beat heart rate), the duration of the systole, the duration of the diastole, the duration of the systolic rise, the duration of the systolic decay, and/or the duration of the entire decay.

Another method to detect an irregular cardiac cycle is to analyze the location of the dichrotic notches of an arterial waveform/signal. For example, the location of a dichrotic notch versus the maximum systolic pressure and the location of a dichrotic notch versus the diastolic pressure (the minimum pressure of the cardiac cycle before the maximum systolic pressure) are analyzed. Additionally, the difference between a value corresponding to the diastolic pressure of the beginning of a cardiac cycle and a value corresponding to a maximum pressure of the cardiac cycle can be used. A further waveform characteristic includes the difference between a value corresponding to the maximum pressure of a cardiac cycle and a value corresponding to the diastolic pressure at the end of a cardiac cycle.

To detect an irregular cardiac cycle, the statistical characteristics, *i.e.,* statistical moments, of the different portions of an arterial waveform as just described are compared. In the methods described herein the first four statistical moments, *i.e.,* mean, variance, skewness, and kurtosis, are used. The following equations can be used to calculate the first four statistical moments (where N is the total number of samples during systole):

Mean:

$$(\text{Equation 6}) \qquad \mu_{1p} = \frac{1}{N-1} \sum_{k=0}^{N-1} P(k)$$

Variance:

$$(\text{Equation 7}) \qquad \mu_{2p} = \sigma_p^2 = \frac{1}{N-1} \sum_{k=0}^{N-1} \left( P(k) - P_{avg} \right)^2$$

Skewness:

$$(\text{Equation 8}) \qquad \mu_{3p} = \frac{1}{N-1} \sum_{k=0}^{N-1} \left( \frac{P(k) - P_{avg}}{\sigma_p} \right)^3$$

Kurtosis:

$$(\text{Equation 9}) \qquad \mu_{4p} = \frac{1}{N-1} \sum_{k=0}^{N-1} \left( \frac{P(k) - P_{avg}}{\sigma_p} \right)^4$$

Additional characteristics that can be used to compare cardiac cycles include the power of the phases of the cardiac cycles as discussed above as well as frequency characteristics and time-frequency characteristics of the phases. The power of a phase of the cardiac cycle is measured as the integral of the cardiac signal under each phase. The power can be calculated by integrating the signal within each phase. Thus, for example, the power of the systole phase, $E_{sys}$, can be calculated using the following equation (where N is the total number of samples during systole):

$$(\text{Equation 10}) \qquad E_{sys} = \sum_{k=0}^{k=N-1} P(k)$$

The frequency characteristics of each phase of a cardiac cycle can be derived by performing a Fourier transform analysis. Various known Fourier transforms including fast Fourier transforms can be used.

The time-frequency characteristics of each phase of a cardiac cycle can be derived using wavelet transform analysis. Wavelet analysis is well suited for analyzing signals which have transients or other non-stationary characteristics in the time domain. In contrast to Fourier transforms, wavelet analysis retains information in the time domain, *i.e.,* when the event occurred.

Waveform characteristics also can be calculated using mathematical interpolation, curve fitting methods, or approximating functions. Examples of mathematical interpolation include spline interpolation, polynomial interpolation, exponential interpolation, linear interpolation, nonlinear interpolation, piecewise interpolation, and multivariate interpolation.

In comparing statistical or other characteristics or parameters of one or more portions of a cardiac cycle to a control cardiac cycle, different approaches can be used. For example, one or more characteristics of a cardiac cycle can be compared to the same characteristic(s) of the cardiac cycle immediately preceding the cardiac cycle being examined, *i.e.,* the control cardiac cycle is the cardiac cycle immediately preceding the cardiac cycle being examined. Another comparison can involve comparing one or more characteristics of a cardiac cycle with the same characteristic(s) of the cardiac cycle immediately following the cardiac cycle being examined, *i.e.,* the control cardiac cycle is the cardiac cycle immediately following the cardiac cycle being examined. A further comparison can involve comparing one or more characteristics of a cardiac cycle with both the cardiac cycle immediately preceding the cardiac cycle being examined and the cardiac cycle immediately following the cardiac cycle being examined, *i.e.,* the control cardiac cycles are the cardiac cycle immediately preceding the cardiac cycle being examined and the cardiac cycle immediately following the cardiac cycle being examined. An additional comparison can involve comparing one or more characteristics of a cardiac cycle with the same characteristic(s) in a median (or mean) cardiac cycle from a sequence containing at least three cardiac cycles, *i.e.,* the control cardiac cycle is a median (or mean) cardiac cycle from a sequence containing at least three cardiac cycles. Another comparison can involve comparing one or more characteristics of a cardiac cycle with the same characteristic(s) in a statistical measurement of a phase of a cardiac cycle, *i.e.,* the control cardiac cycle is a statistical representation of the measurement being compared. These comparison examples have been presented as comparisons of one or more characteristics, however, as will be apparent to one of skill in the art, multiple parameters for individual or multiple portions of the cardiac cycle can be used. Further, as will also be apparent to one of skill in the art, as these methods are likely to be performed using computer devices, a large number of these comparisons can be performed in real time.

In making such comparisons, predetermined thresholds can be used. As used herein, a predetermined threshold is a value assigned prior to a comparison being made. Generally, the predetermined threshold for a parameter will indicate a value related to a control cardiac cycle as measured, for example, from the subject being monitored, from averaged, or from anthropomorphic data. Depending on the parameter measured, the predetermined threshold can be a very small value or difference, or could be a larger value. Such predetermined thresholds will be easily provided by a medical professional or instrument operator. The predetermined threshold amount selected for a particular parameter will depend on the accuracy of the particular parameter used.

For example, if a single parameter is used, a predetermined threshold amount can be a difference of 30 percent or more as compared to the same parameter of the control cardiac cycle, a difference of 25 percent or more as compared to the same parameter of the control cardiac cycle, a difference of 20 percent or more as compared to the same parameter of the control cardiac cycle, a difference of 15 percent or more as compared to the same parameter of the control cardiac cycle, a difference of 10 percent or more as compared to the same parameter of the control cardiac cycle, a difference of 5 percent or more as compared to the same parameter of the control cardiac cycle, a difference of 4 percent or more as compared to the same parameter of the control cardiac cycle, a difference of 3 percent or more as compared to the same parameter of the control cardiac cycle, a difference of 2 percent or more as compared to the same parameter of the control cardiac cycle, a difference of 1 percent or more as compared to the same parameter of the control cardiac cycle, a difference of 0.5 percent or more as compared to the same parameter of the control cardiac cycle, a difference of 0.4 percent or more as compared to the same parameter of the control cardiac cycle, a difference of 0.3 percent or more as compared to the same parameter of the control cardiac cycle, a difference of 0.2 percent or more as compared to the same parameter of the control cardiac cycle, or a difference of 0.1 percent or more as compared to the same parameter of the control cardiac cycle.

Further, if more than one parameter is used, the predetermined threshold amount will depend on the particular combination of parameters used in combination with the accuracy of the parameter measurements. For example, if more than one parameter is used, a predetermined threshold amount can be a difference of 30 percent or more as compared to the same one or more parameters of the control cardiac cycle, a difference of 25 percent or more as compared to the same one or more parameters of the control cardiac cycle, a difference of 20 percent or more as compared to the same one or more parameters of the control cardiac cycle, a difference of 15 percent or more as compared to the same one or more parameters of the control cardiac cycle, a difference of 10 percent or more as compared to the same one or more parameters of the control cardiac cycle, a difference of 5 percent or more as compared to the same one or more parameters r of the control cardiac cycle, a difference of 4 percent or more as compared to the same one or more parameters of the control cardiac cycle, a difference of 3 percent or more as compared to the same one or more parameters of the control cardiac cycle, a difference of 2 percent or more as compared to the same one or more parameters of the control cardiac cycle, a difference of 1 percent or more as compared to the same one or more parameters of the control cardiac cycle, a difference of 0.5 percent or more as compared to the same one or more parameters of the control cardiac cycle, a difference of 0.4 percent or more as compared to the same parameter of the control cardiac cycle, a difference of 0.3 percent or more as compared to the same parameter of the control cardiac cycle, a difference of 0.2 percent or more as compared to the same parameter of the control cardiac cycle, or a difference of 0.1 percent or more as compared to the same parameter of the control cardiac cycle.

In addition to the predetermined thresholds, all the parameters used for an analysis can be assembled in a single parameters data set. In a dataset, the accuracy of a particular parameter defines the weight of the parameter in the parameters data set. Based on the weight of a respective parameter in the parameters dataset, a threshold is assigned to each parameter and the number of parameters from the parameters dataset exceeding the predetermined thresholds is counted. When multiple parameters are used, each parameter can have its own predetermined threshold amount. For example, a predetermined threshold amount can be a difference of 30 percent or more as compared to the same parameter of the control cardiac cycle, a difference of 25 percent or more as compared to the same parameter of the control cardiac cycle, a difference of 20 percent or more as compared to the same parameter of the control cardiac cycle, a difference of 15 percent or more as compared to the same parameter of the control cardiac cycle, a difference of 10 percent or more as compared to the same parameter of the control cardiac cycle, a difference of 5 percent or more as compared to the same parameter of the control cardiac cycle, a difference of 4 percent or more as compared to the same parameter of the control cardiac cycle, a difference of 3 percent or more as compared to the same parameter of the control cardiac cycle, a difference of 2 percent or more as compared to the same parameter of the control cardiac cycle, a difference of 1 percent or more as compared to the same parameter of the control cardiac cycle, or a difference of 0.5 percent or more as compared to the same parameter of the control cardiac cycle. As a specific example, a first parameter could have a predetermined threshold amount of a difference of 15 percent or more as compared to the same parameter of the control cardiac cycle and a second parameter could have a predetermined threshold amount of a difference of 4 percent or more as compared to the same parameter of the control cardiac cycle. The number of predetermined threshold amounts can be equal to or less than the number of parameters evaluated.

The respiratory factor can be used along with the regular cardiac cycles to estimate waveform characteristics that are used to replace the waveform characteristics of irregular cardiac cycles in the modified waveform. The respiratory parameter is a respiratory-induced variation in the waveform dataset caused by respiration, e.g., spontaneous breathing or mechanical ventilation. Examples of mechanical ventilation include pressure controlled ventilation, volume controlled ventilation, synchronized intermittent mandatory ventilation, pressure support ventilation, volume support ventilation, high frequency ventilation, synchronized intermittent positive pressure ventilation, continuous positive pressure ventilation, and non-invasive ventilation. Fig. 8 shows a waveform 800 for an arterial blood pressure signal with no irregular cardiac cycles. A respiratory parameter 810 for this regular waveform, which was calculated based on the waveform characteristic of systolic pressure, is shown by a dashed line that roughly follows the systolic (maximal) pressure for each cardiac cycle. The respiratory parameter can relate to other features of the waveform such as the minimal pressure, the diastolic pressure, the dichrotic notch, the standard deviation of the cardiac cycles, or the ending or starting points of each cardiac cycle. The respiratory parameter can represent multiple characteristics, for example, the respiration function can include the maximal pressure and the difference between the minimum and maximum. The use of systolic pressure, for example, provides a maximal alignment point for the replacement waveform characteristics when the waveform characteristics of an irregular cardiac cycle such as a missing cardiac cycle are replaced.

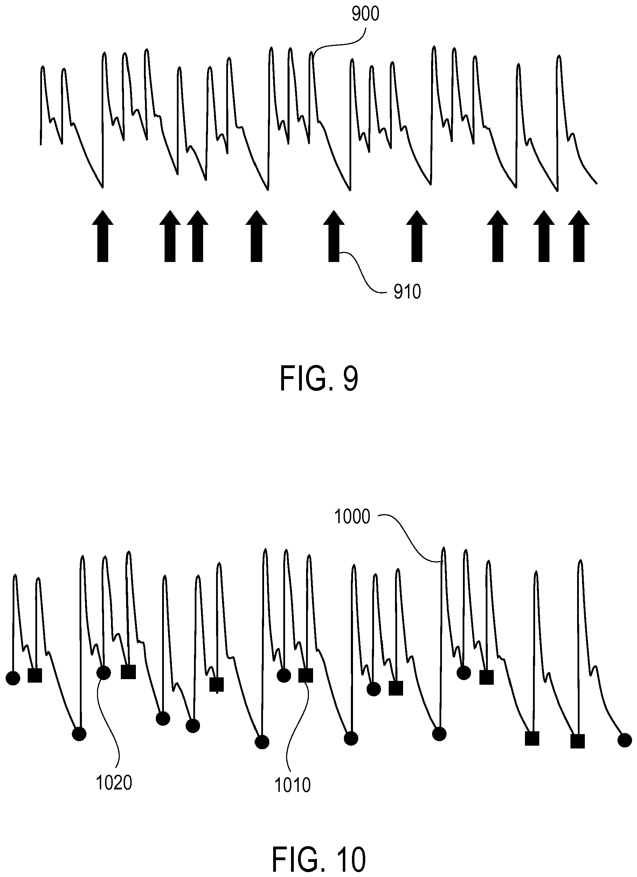

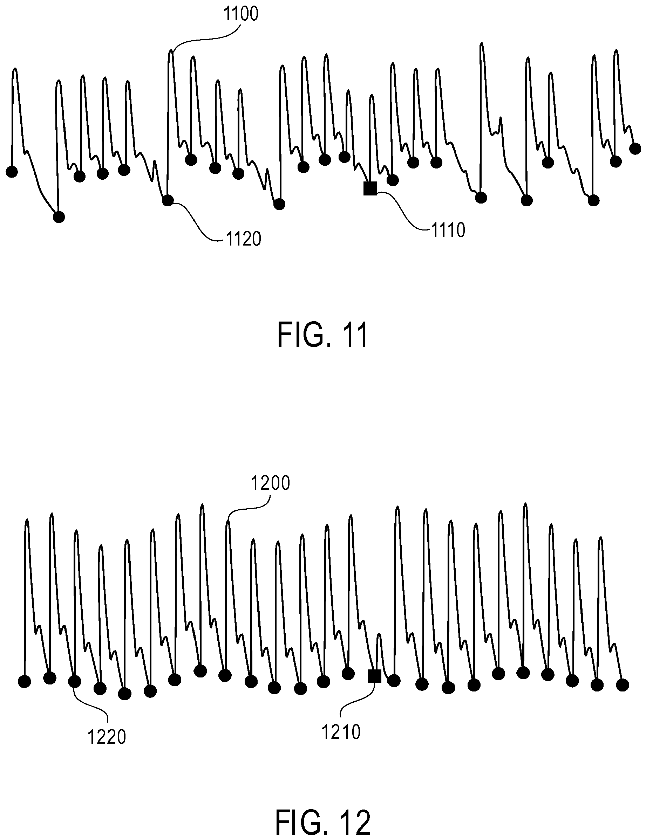

Some of the waveform signal irregularities that are accounted for using the methods described herein can be illustrated by the following examples. Fig. 9 shows a waveform 900 for an arterial blood pressure signal in which irregular cardiac cycles are indicated with arrows 910. If the waveform characteristics of these irregular cardiac cycles as well as their following cardiac cycles are not replaced with estimated values, the calculated stroke volume variation, for example, will be larger than its true value. Fig. 10 shows a different waveform 1000 for an arterial blood pressure signal showing some irregular cardiac cycles. The starting point of exemplary irregular cardiac cycles is indicated with a square 1010 (and the starting points of other cardiac cycles are indicated with a circle 1020). One way to identify the indicated irregular cardiac cycles is to use the method described above of monitoring the pulse rate, i.e., the irregular beats have longer durations than the regular beats. Fig. 11 shows a further waveform 1100 for an arterial blood pressure signal in which the starting point of an irregular cardiac cycle is indicated with a square 1110 (and the starting points of other cardiac cycles are indicated with a circle 1120). This irregular cardiac cycle can be detected, for example, by monitoring systolic pressure, i.e., this irregular cardiac cycle has a lower systolic pressure than the regular cardiac cycles. Fig. 12 shows another waveform 1200 for an arterial blood pressure signal in which the starting point of the irregular cardiac cycle is indicated with a square 1210 (and the starting points of other cardiac cycles are indicated with a circle 1220). This irregular cycle can be detected, for example, by monitoring pulse pressure, i.e., the irregular cardiac cycle has a lower pulse pressure than the regular cardiac cycles. Pulse pressure (PP) as used herein is systolic pressure ($P_{sys}$) minus diastolic pressure ($P_{dia}$) or $PP = P_{sys} - P_{dia}$.

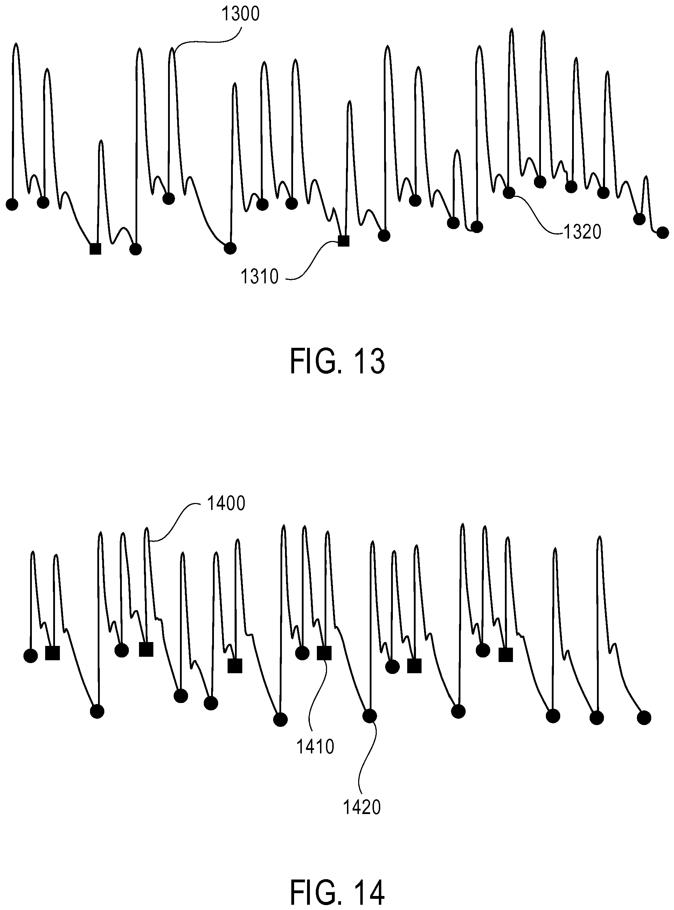

Monitoring single parameters as described for Figs. 9-12 will find many irregular cardiac cycles, however multiple parameters also can be monitored. For example, Fig. 13 shows a waveform 1300 for an arterial blood pressure signal in which the starting points of two irregular cardiac cycles are indicated with squares 1310 (and the starting points of other cardiac cycles are indicated with a circle 1320). These irregular cardiac cycles can be identified, for example, by monitoring both systolic pressure and pulse rate, i.e., both these cardiac cycles have smaller systolic pressure than the regular beats as well as having longer durations. Fig. 14 shows a further waveform 1400 for an arterial blood pressure signal in which the starting points of multiple irregular cardiac cycles are indicated with squares 1410 (and the starting points of other cardiac cycles are indicated with a circle 1420). These indicated irregular cardiac cycles have different left-side pulse pressure than right-side pulse pressure and can be identified by looking at these values. As used herein Left-side Pulse Pressure (LPP) is the systolic pressure of the current cardiac cycle ($P_{sys}$) minus the diastolic pressure of the current cardiac cycle ($P_{dia\_cur}$) or LPP = $P_{sys}$ - $P_{dia\_cur}$, and Right-side Pulse Pressure (RPP) is the systolic pressure of the current cardiac cycle ($P_{sys}$) minus the diastolic pressure of the next cardiac cycle ($P_{dia\_next}$) or RPP = $P_{sys}$ - $P_{dia\_next}$. By using one or more of these methods the irregular cardiac cycles can be identified and their adverse impact on the calculation of the cardiac parameter minimized.

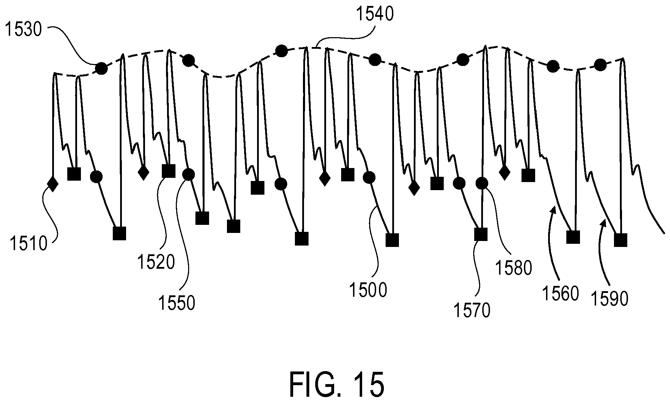

Fig. 15 can be used to explain how the waveform characteristics of some irregular cardiac cycles can be restored and replaced with estimated waveform characteristics. In Fig. 15, waveform 1500 is an arterial blood pressure signal with both regular and irregular cardiac cycles. In waveform 1500, the starting point of each regular cardiac cycle 1510 is indicated with a diamond, and the starting point of each irregular cycle 1520 is indicated with a square. Some cardiac cycles are missing from the waveform due to the effect of irregular cardiac cycles. The location of the systolic pressure 1530 of such a missing cycle is indicated by a solid circle on the respiratory parameter 1540.

Some irregular cardiac cycles have longer durations than regular cycles, but, aside from the extended duration, these irregular cardiac cycles have directly measurable waveform parameters, such as systolic pressure, that are not abnormal and can be directly used in calculations. To use the waveform parameters of these irregular cardiac cycles, a pseudo-starting and/or pseudo-ending point is determined. To determine pseudo-starting points and/or pseudo-ending points a time factor based on all the regular cardiac cycles in a given waveform dataset is first determined. The time factor can be, for example, the median or mean time duration for the regular cycles. Then a pressure factor based on the diastolic pressures of the cycles is calculated for each regular cycle. The pressure factor can be, for example, a maximum pressure difference determined by finding the maximum difference in pressure between each cycle's diastolic pressure and the next cycles' diastolic pressure. Next, for an irregular cardiac cycle with an ending point that is bad (such as cycle 1520 in figure 15), if its starting point is determinable, the pressure data for the waveform that is in a pre-established range from the starting point is examined. The pre-established range from the starting point can be, for example, the starting point plus (a range start factor * the median time duration) and the starting point plus (a range stop factor * the median time duration). Examples of range start factors include, but are not limited to, 0.5, 0.55, 0.6, 0.65, 0.7, 0.75, 0.8, 0.85, 0.86, 0.87, 0.88, 0.89, 0.9, 0.91, 0.92, 0.93, 0.94, 0.95, 0.96, 0.97, 0.98, and 0.99. Examples of range stop factors include, but are not limited to, 1.01, 1.02, 1.03, 1.04, 1.05, 1.06, 1.07, 1.08, 1.09, 1.1, 1.11, 1.12, 1.13, 1.14, 1.15, 1.2, 1.25, 1.3, 1.35, 1.4, 1.45, and 1.5. A further example of a pre-established range from the starting point is the range of the starting point plus (0.9 * the median time duration) to the starting point plus (1.1 * the median time duration). Additionally, the pre-established range from the staring point can be determined based on the standard deviation of the duration, i.e., the pre-determined range from the staring point can be the starting point plus (the median duration minus the standard deviation of the duration) to starting point plus (the median duration plus the standard deviation of the duration). Assuming there is a pressure data point within the pre-established range, if the relationship between the pressure data point and the pressure factor is within a predetermined range, that point is assigned to be the pseudo-ending point. For example, if the difference in pressure between that pressure data point and the starting point's pressure is smaller than the maximum difference, that point could be assigned to be the pseudo-ending point. If there is more than one point satisfying this criteria, and the relationship between each of the more than one pressure data points and the pressure factor is within a predetermined range, the pressure data point with a time value that is closest to the time factor is assigned to be the pseudo-ending point. For example, if the difference in time between the more than one pressure data points and the starting point is smaller than a maximum time difference the pressure data point with a time that is closest to the starting point plus the median duration could be assigned to be the pseudo-ending point. If there is no pressure data within the pre-established range or the relationship between the pressure data points and the pressure factor is outside the predetermined range, a pseudo-ending point cannot be calculated reliably and no pseudo-ending point is assigned. For example, if the difference in pressure between the found pressure and the starting point's pressure is larger than a maximum pressure difference, a pseudo-ending point could not assigned.

For an irregular cardiac cycle with a bad starting point (such as cycle 1570 in figure 15), if its ending point is determinable, the pressure data that is in a pre-established range from the ending point is examined. The pre-established range from the ending point can be, for example, the ending point minus (a range begin factor * the median time duration) and the ending point minus (a range end factor * the median time duration). Examples of range begin factors include, but are not limited to, 1.01, 1.02, 1.03, 1.04, 1.05, 1.06, 1.07, 1.08, 1.09, 1.1, 1.11, 1.12, 1.13, 1.14, 1.15, 1.2, 1.25, 1.3, 1.35, 1.4, 1.45, and 1.5. Examples of range end factors include, but are not limited to, 0.5, 0.55, 0.6, 0.65, 0.7, 0.75, 0.8, 0.85, 0.86, 0.87, 0.88, 0.89, 0.9, 0.91, 0.92, 0.93, 0.94, 0.95, 0.96, 0.97, 0.98, and 0.99. A further example of a pre-established range from the ending point is the range of the ending point minus (1.1 * the median time duration) to the ending point minus (0.9 * the median time duration). Additionally, the pre-established range from the ending point can be determined based on the standard deviation of the duration, i.e., the pre-determined range from the ending point can be the starting point minus (the median duration plus the standard deviation of the duration) to starting point minus (the median duration minus the standard deviation of the duration). Assuming there is a pressure data point within pre-established range, if the relationship between the pressure data point and the pressure factor is within a predetermined range, that point is assigned to be the pseudo-starting point. For example, if the difference in pressure between that pressure data point and the ending point's pressure is smaller than the maximum difference, that point could be assigned to be the pseudo-starting point. If there is more than one point satisfying this criteria, and the relationship between each of the more than one pressure data points and the pressure factor is within a predetermined range, the pressure data point with a time value that is closest to the time factor is assigned to be the pseudo-ending point. For example, if the difference in time between the more than one pressure data points and the ending point is smaller than a maximum time difference the pressure data point with a time that is closest to the ending point plus the median duration could be assigned to be the pseudo-ending point. If there is no pressure data within the pre-established range or the relationship between the pressure data points and the pressure factor is outside the predetermined range, a pseudo-starting point cannot be calculated reliably and no pseudo-starting point is assigned. For example, if the difference in pressure between the found pressure and the ending point's pressure is larger than a maximum pressure difference, a pseudo-starting point could not assigned.

For an irregular cardiac cycle whose starting and ending points are both bad (such as the cardiac cycle at 1590), an estimated starting point can be used, such as a point that has the same pressure as its nearest neighbor cycle's starting point, then the pseudo-ending point can be estimated as described above. An estimated ending point is then used to determine a pseudo-starting point. The estimated ending point has the same pressure as its nearest neighbor cycle's ending point, and the pseudo-starting point is calculated as described above. Only when the estimated starting point and the calculated pseudo-starting point and the estimated ending point and the calculated pseudo-ending point respectively are close to each other are the pseudo-starting point and pseudo-ending point adopted for the purpose of further calculations; otherwise the data is too variable and the pseudo-starting and pseudo-ending points cannot be determined reliably. To be considered close to each other, the two points are within a predetermined time of each other. The length of the predetermined time will be set by one of skill in the art to provide an acceptable level of confidence that the pseudo-starting and pseudo-ending points will have the accuracy desired for the calculations to be performed using the data. Examples of such a predetermined time include 5 seconds, 4 seconds, 3 seconds, 2 seconds, 1 seconds, 0.5 seconds, 0.4 seconds, 0.3 seconds, 0.2 seconds, 0.1 seconds, 0.05 seconds, 0.04 seconds, 0.03 seconds, 0.02 seconds, or 0.01 seconds.

An example of such an irregular cardiac cycle is shown in Fig. 15 by the irregular cycle starting at point 1520. The pseudo-ending point for this irregular cardiac cycle can be determined as just discussed based on the waveform characteristics of regular cardiac cycles to be pseudo-ending point 1550. Similarly, the pseudo-starting point of a further irregular cardiac cycle that begins at point 1570 is calculated to be pseudo-starting point 1580. Once the pseudo-starting and/or pseudo-ending points are calculated for such irregular cardiac cycles as discussed above, the directly measured waveform parameters for those cardiac cycles and calculable waveform parameters/characteristics based on the directly measured waveform parameters can be used in further calculations with the waveform parameters/characteristics of regular cardiac cycles. However, as discussed above the pseudo-starting point and/or the pseudo-ending point of irregular cardiac cycles cannot always be determined reliably so the waveform parameters/characteristics of some irregular cardiac cycles are not able to be determined this way or further used in calculations. For example, the pseudo-ending point of the irregular cardiac cycle indicated at 1560 cannot be determined reliably, i.e., the point found between starting point plus (0.9 * the median time duration) and the starting point plus (1.1 * the median time duration) did not meet the criteria discussed above. Note that calculated waveform parameters such as pulse pressure and standard deviation of the cycle need to be recalculated once the pseudo-starting and/or pseudo-ending points are determined.

The estimated or calculated waveform characteristics for those irregular cardiac cycles which contain determinable cardiac parameters can be directly computed from the determined parameters. For all other irregular cardiac cycles, including missing cardiac cycles, waveform characteristics can be estimated based on the waveform characteristics of the regular cardiac cycles and the respiratory parameter 1540. The respiratory parameter 1540 was calculated in this example based on the systolic pressure of the regular cardiac cycles and the systolic pressure of some irregular cardiac cycles, and it provides a relative placement point for estimated waveform characteristics, i.e., once pressure related waveform characteristics are estimated they can be adjusted relative to the respiratory parameter. The estimated waveform characteristics are then used to replace the original waveform characteristics for the irregular cardiac cycles in the modified waveform.

One example of the calculation of such waveform characteristics is the determination of the standard deviation of an irregular cycle ($\sigma_{irregular\_cycle}$) which can be calculated using Equation 11:

$$(\text{Equation 11})\ \sigma_{irregular\_cycle} = \left( \frac{P_{sys(irregular\_cycle)}}{P_{sys(nearest\_regular\_cycle)}} \right) \times \sigma_{nearest\_regular\_cycle}$$

In Equation 11, the systolic pressure ($P_{sys(nearest\_regular\_cycle)}$) and the standard deviation ($\sigma_{(nearest\_regular\_cycle)}$) of the nearest regular cycle are known, and the systolic pressure of the irregular cycle ($P_{sys(irregular\_cycle)}$) is known from the respiratory parameter. Note the systolic pressure can be replaced in the equation for other parameters such as pulse pressure or other waveform characteristics to provide the standard deviation of that parameter in the irregular cycle. Additionally, the relationship between parameters can be linear, i.e., y=a*x+b, instead of y=a*x as shown in Equation 11, where y is the standard deviation, x is the systolic pressure, and a and b are constants. Other mathematical relationships such as non-linear, polynomial, or others can also be used depending on the determined relationship between the parameters. In some situations, there may be two nearest cycles (i.e., one before and one after) in which case both will be used and the final standard deviation of the irregular cycle will be the average of the two calculated independently. Additionally, cycles other than the nearest cycles may also be used, as the relationship between the standard deviation and a waveform characteristic can be obtained from regular cycles. As an example, standard deviation (y) and systolic pressure (x) can be linearly related, e.g., y=a*x+b, and if a and b are determined first from the y and x of regular cycles, then y of the irregular cycle can be calculated given its systolic pressure.

Creating a modified waveform that accounts for irregular cardiac cycles as described herein increases the accuracy and sensitivity of calculations performed on the dataset. Therefore, calculations such as stroke volume variation, pulse pressure variation, systolic pressure variation, stroke volume, cardiac output, systemic vascular compliance, cardiac flow, cardiac flow velocity, vascular compliance, and/or vascular elastance achieve increased accuracy and sensitivity. An example of a ventricular stroke volume variation calculation is provided in U.S. Patent Application Publication No. US 2005/0187481, which is incorporated by reference herein in its entirety.

In addition to creating a modified waveform dataset, other operations can be performed on the dataset prior to or after modification to increase the accuracy and sensitivity of calculations performed using the waveform dataset. For example, the signal can be filtered to reduce the effect of noise, interference, and artifacts that may occur in the signal. Such filtering can be accomplished through the use of a low-pass filter for example. Following filtering, large motion artifacts can be detected and removed from the waveform dataset. Such artifacts are common as they often result from patient movement, electrical interference, signal noise, or from flushing of an arterial line. Additionally, bad cardiac cycles can be removed after beat detection before detecting irregular cardiac cycles.

Once identified, an irregular cardiac cycle for which the waveform characteristics have been replaced can be indicated on a graphical user interface. When the waveform dataset corresponding to an arterial blood pressure, or any signal proportional to or derived from the arterial pressure signal, such as pulseox, Doppler ultrasound, or bioimpedance signal is displayed on a graphical user interface simultaneously with the steps of the methods described herein, indications that irregular cardiac cycles are present generally or a specific indication that a particular cardiac cycle is an irregular cardiac cycle can be provided. The same information can be provided for data not shown in real time.

The time period for the waveform dataset can be a set value, for example, the time period can be about ten minutes or more, about five minutes of more, about four minutes or more, about three minutes or more, about two minutes or more, about one minute or more, about 50 seconds or more, about 40 seconds or more, about 30 seconds or more, about 20 seconds or more, about 10 seconds or more, about 5 seconds or more, about 2 seconds or more, about 1 second or more, about 0.5 second or more, or about 0.1 second or more. For example, the time period can be about ten, about nine, about eight, about seven, about six, about five, about four, about three, about two, or about one minutes. Further, for example, the time period can be about 55, about 50, about 45, about 40, about 35, about 30, about 25, about 20, about 15, about 10, about 5, about 2, about 1, about 0.5, or about 0.1 seconds. This time period can be constant or can be variable. Further, if irregular cardiac cycles are detected, the time period for the waveform dataset can be increased or decreased. Such an increase or decrease in sample time may improve detection ability and the consistency of the data.

As used herein the term "arterial blood pressure" refers to the force exerted by circulating blood on the walls of blood vessels and an "arterial blood pressure signal" is a signal from a blood pressure monitoring instrument such as a sphygmomanometer or other pressure transducer. As used herein the term "pulseox" refers to a signal from a pulse oximeter, which is an instrument that indirectly measures the amount of oxygen in a subject's blood using using various characteristics of light absorption. As used herein the term "bioimpedance signal" refers to a signal from a bioimpedance plethysmography device, *i.e.,* a device that measures blood parameters such as pulsatile blood volume changes in the aorta. As used herein, the term "Doppler ultrasound" refers to a signal from a Doppler ultrasound device, a device that makes Doppler enhanced ultrasound measurements.

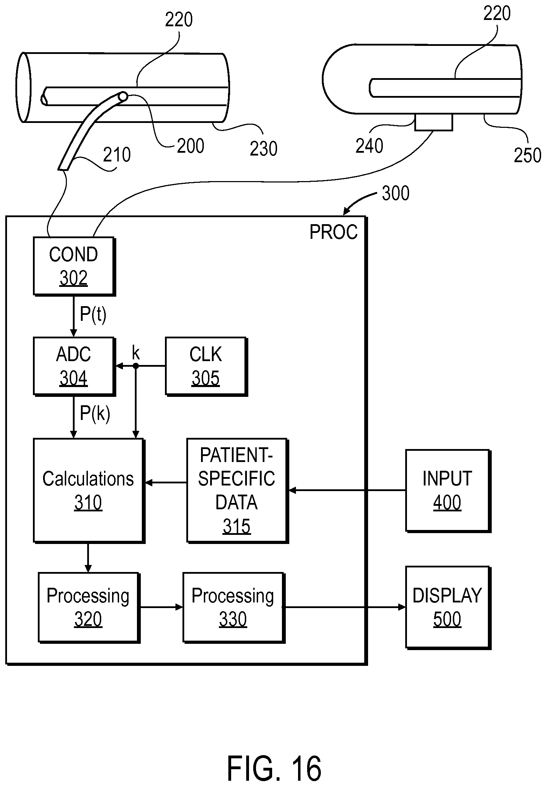

Fig. 16 shows the main components of a system that can be used to implement the methods described herein for determining a cardiovascular parameter. The methods may be implemented within an existing patient-monitoring device, or it may be implemented as a dedicated monitor. As is mentioned above, an arterial blood pressure signal related waveform, or some other input signal proportional to, derived from, or a function of arterial blood pressure signal, may be sensed in either or, indeed, both, of two ways: invasively and non-invasively. For convenience, the system is described as measuring arterial blood pressure.

Fig. 16 shows both invasive and non-invasive types of pressure sensing for the sake of completeness. In most practical applications of the methods described herein, either one or several variations will typically be implemented. In invasive applications of the methods described herein, a conventional pressure sensor 200 is mounted on a catheter 210, which is inserted in an artery 220 of a portion 230 of the body of a human or animal patient. The artery 220 is any artery in the arterial system, such as, for example, the femoral, radial or brachial artery. In the non-invasive applications of the methods described herein, a conventional pressure sensor 240, such as a photo-plethysmographic blood pressure probe, is mounted externally in any conventional manner, for example using a cuff around a finger 250 or a transducer mounted on the wrist of the patient. Fig. 16 schematically shows both types.

The signals from the sensors 200, 240 are passed via any known connectors as inputs to a processing system 300, which includes one or more processors and other supporting hardware and system software (not shown) usually included to process signals and execute code. The methods described herein may be implemented using a modified, standard, personal computer, or may be incorporated into a larger, specialized monitoring system. For use with the methods described herein, the processing system 300 also may include, or is connected to, conditioning circuitry 302 which performs normal signal processing tasks such as amplification, filtering, or ranging, as needed. The conditioned, sensed input pressure signal P(t) is then converted to digital form by a conventional analog-to-digital converter ADC 304, which has or takes its time reference from a clock circuit 305. As is well understood, the sampling frequency of the ADC 304 should be chosen with regard to the Nyquist criterion so as to avoid aliasing of the pressure signal (this procedure is very well known in the art of digital signal processing). The output from the ADC 304 will be the discrete pressure signal P(k), whose values may be stored in conventional memory circuitry (not shown).

The values P(k) are passed to or accessed from memory by a software module 310 comprising computer-executable code for implementing one or more aspects of the methods as described herein. The design of such a software module 310 will be straight forward to one of skill in the art of computer programming. Additional processing as used by a method can be performed in additional modules such as 320 and 330.

If used, signal-specific data such as a previously determined starting time point or a previously determined end time point or other patient-specific data can be stored in a memory region 315, which may also store other predetermined parameters as needed. These values may be entered using any known input device 400 in the conventional manner.

As illustrated by Fig. 16, the results may be ultimately displayed on a conventional display or recording device 500 for presentation to and interpretation by a user. As with the input device 400, the display 500 will typically be the same as is used by the processing system for other purposes.

Exemplary embodiments of the present invention have been described above. One of skill in the art will understand that each step of the methods can be implemented by various means including computer program instructions. These computer program instructions may be loaded onto a general purpose computer, special purpose computer, or other programmable data processing apparatus to produce a machine, such that the instructions which execute on the computer or other programmable data processing apparatus create a means for implementing the functions specified in the methods.

The methods described herein further relate to computer program instructions that may be stored in a computer-readable memory that can direct a computer or other programmable data processing apparatus, such as in a processor or processing system (shown as 300 in Fig. 16), to function in a particular manner, such that the instructions stored in the computer-readable memory produce an article of manufacture including computer-readable instructions for implementing the function specified in the blocks illustrated in Fig. 16. The computer program instructions may also be loaded onto a computer, the processing system 300, or other programmable data processing apparatus to cause a series of operational steps to be performed on the computer, the processing system 300, or other programmable apparatus to produce a computer-implemented process such that the instructions that execute on the computer or other programmable apparatus provide steps for implementing the functions specified in the blocks. Moreover, various software modules 310, 320, and 330 can be used to perform the various calculations and perform related method steps described herein also can be stored as computer-executable instructions on a computer-readable medium in order to allow the methods to be loaded into and executed by different processing systems.

Steps of the methods described herein can be implemented by combinations of means for performing the specified functions, combinations of steps for performing the specified functions, and program instruction means for performing the specified functions. One of skill in the art will understand that each method step can be implemented by special purpose hardware-based computer systems that perform the specified functions or steps, or combinations of special purpose hardware and computer instructions.

The methods disclosed herein are equally applicable to any subject for which an arterial blood pressure, pulseox, Doppler ultrasound, or bioimpedance signal can be detected. For example, the subject can be, but is not limited to a mammal such as a human.

The present claims are not limited in scope by the embodiments disclosed herein which are intended as illustrations of a few aspects of the invention and any embodiments which are functionally equivalent are within the scope of the claims. Various modifications of the methods in addition to those shown and described herein will become apparent to those skilled in the art and are intended to fall within the scope of the claims. Further, while only certain representative combinations of the method steps disclosed herein are specifically discussed in the embodiments above, other combinations of the method steps will become apparent to those skilled in the art and also are intended to fall within the scope of the claims. Thus a combination of steps may be explicitly mentioned herein; however, other combinations of steps are included, even though not explicitly stated. The term "comprising" and variations thereof as used herein is used synonymously with the term "including" and variations thereof and are open, non-limiting terms.

**Claims**

1. A computer-implemented method of determining a cardiovascular parameter comprising the steps:

   receiving a waveform dataset corresponding to an arterial blood pressure, or a signal proportional to, or derived from, the arterial blood pressure signal;
   identifying individual cardiac cycles in the waveform dataset;
   measuring waveform characteristics for the individual cardiac cycles;
   determining if the individual cardiac cycles are regular cardiac cycles or irregular cardiac cycles;
   **characterized in that**
   measuring a respiratory parameter which is a respiratory-induced variation in the waveform dataset caused by respiration;
   creating a modified waveform dataset containing the waveform characteristics of the regular cardiac cycles and the waveform characteristics of the irregular cardiac cycles wherein the waveform characteristics of the irregular cardiac cycles are replaced with estimated waveform characteristics, wherein replacing the waveform characteristics of the irregular cardiac cycles with estimated waveform characteristics comprises:
   determining, based on the waveform characteristics of regular cardiac cycles, pseudo-starting points and/or pseudo-ending points for the irregular cardiac cycles which have longer durations than regular cycles, and directly measuring or calculating waveform parameters/characteristics of the irregular cardiac cycle based on the pseudo-starting points and/or pseudo-ending points; and
   calculating a cardiovascular parameter using the modified waveform dataset, wherein the cardiovascular parameter is a parameter reflecting preload dependence and fluid responsiveness, such as stroke volume variation, pulse pressure variation, systolic pressure variation, stroke volume, cardiac output, systemic vascular compliance, cardiac flow, cardiac flow velocity, vascular compliance, or vascular elastance.

2. The computer-implemented method of claim 1, wherein determining if the individual cardiac cycles are regular cardiac cycles or irregular cardiac cycles comprises:

   comparing one or more waveform characteristics of the individual cardiac cycle to one or more waveform characteristics of a control cardiac cycle; and
   identifying the individual cardiac cycle as an irregular cardiac cycle if the one or more waveform characteristics of the individual cardiac cycle differs from the one or more waveform characteristics of the control cardiac cycle by a predetermined threshold amount.

3. The computer-implemented method of claim 1, wherein determining the pseudo-ending point for an irregular cycle comprises:

   determining a time factor for all the regular cardiac cycles;
   determining a pressure factor based on the diastolic pressures of the cycles;
   identifying a starting point of the irregular cycle and pressure data points that are within a pre-established range from the starting point;
   if there is a pressure data point within the pre-established range and the relationship between the pressure data point and the pressure factor is within a predetermined range, that pressure data point is assigned to be the pseudo-ending point; and
   if there is more than one pressure data point within the range and the relationship between each of the more than one pressure data points and the pressure factor is within a predetermined range, the pressure data point

EP 2 528 499 B1

with a time value that is closest to the time factor is assigned to be the pseudo-ending point.

4.  The computer-implemented method of claim 3, wherein the pre-established range from the starting point is the starting point plus (a range start factor * the median time duration) to the starting point plus (a range stop factor * the median time duration).

5.  The computer-implemented method of claim 4, wherein the range start factor is 0.9 and the range stop factor is 1.1.

6.  The computer-implemented method of claim 1, wherein determining the pseudo-starting point for an irregular cycle comprises:

> determining a time factor for all the regular cardiac cycles;
> determining a pressure factor based on the diastolic pressures of the cycles;
> identifying an ending point of the irregular cycle and pressure data points for the waveform that are within a pre-established range from the ending point;
> if there is a pressure data point within the pre-established range and the relationship between the pressure data point and the pressure factor is within a predetermined range, that pressure data point is assigned to be the pseudo-starting point; and
> if there is more than one pressure data point within the range and the relationship between each of the more than one pressure data points and the pressure factor is within a predetermined range, the pressure data point with a time value that is closest to the time factor is assigned to be the pseudo-ending point.

7.  The computer-implemented method of claim 6, wherein the pre-established range from the starting point is the starting point plus (a range begin factor * the median time duration) to the starting point plus (a range end factor * the median time duration).

8.  The computer-implemented method of claim 7, wherein the range begin factor is 1.1 and the range end factor is 0.9.

9.  The computer-implemented method of claim 1, wherein the waveform characteristic is the standard deviation of an irregular cycle.

10. The computer-implemented method of claim 9, wherein the standard deviation ($\sigma_{\text{irregular\_cycle}}$) of an irregular cycle is calculated by:

> determining the systolic pressure ($P_{\text{sys(nearest\_regular\_cycle)}}$) of the nearest regular cycle;
> determining the standard deviation ($\sigma_{\text{nearest\_regular\_cycle}}$) of the nearest regular cycle;
> determining the systolic pressure ($P_{\text{sys(irregular\_cycle)}}$) of the irregular cycle based on the respiratory parameter and the systolic pressure ($P_{\text{sys(nearest\_regular\_cycle)}}$) of the nearest regular cycle; and
> solving the equation:

$$\sigma_{irregular\_cycle} = \left( \frac{P_{sys(irregular\_cycle)}}{P_{sys(nearest\_regular\_cycle)}} \right) \times \sigma_{nearest\_regular\_cycle}$$

11. The computer-implemented method of claim 1, further comprising filtering the waveform dataset with a low-pass filter.

12. The computer-implemented method of claim 1, further comprising indicating the position of the estimated cardiac cycles on a graphical user interface.

13. The computer-implemented method of claim 1, further comprising when an irregular cardiac cycle is detected indicating that the waveform contains estimated cardiac cycles on a graphical user interface.

14. The computer-implemented method of claim 1, wherein the waveform dataset is from a sampling period of a set duration.

15. A computer-readable medium storing computer-executable instructions which, when loaded into and executed by

17

a processing system, cause the processing system to carry out the method of any of the previous claims.

**Patentansprüche**

1. Computerimplementiertes Verfahren zum Bestimmen eines Herz-Kreislaufparameters, umfassend die Schritte, dass:

   ein einem Arterienblutdruck entsprechender Wellenformdatensatz oder ein zu dem Arterienblutdrucksignal proportionales oder von diesem abgeleitetes Signal empfangen wird;
   in dem Wellenformdatensatz einzelne Herzzyklen identifiziert werden;
   Wellenformkennlinien für die einzelnen Herzzyklen gemessen werden;
   bestimmt wird, ob es sich bei den einzelnen Herzzyklen um regelmäßige Herzzyklen oder um unregelmäßige Herzzyklen handelt;
   **dadurch gekennzeichnet, dass**
   ein respiratorischer Parameter gemessen wird, bei dem es sich um eine respiratorisch bedingte Schwankung bei dem Wellenformdatensatz handelt, die durch die Atmung verursacht wird;
   ein modifizierter Wellenformdatensatz erzeugt wird, welcher die Wellenformkennlinien der regelmäßigen Herzzyklen und die Wellenformkennlinien der unregelmäßigen Herzzyklen enthält, wobei die Wellenformkennlinien der unregelmäßigen Herzzyklen durch geschätzte Wellenformkennlinien ersetzt werden, wobei das Ersetzen der Wellenformkennlinien der unregelmäßigen Herzzyklen durch geschätzte Wellenformkennlinien umfasst:

   dass basierend auf den Wellenformkennlinien von regelmäßigen Herzzyklen Pseudo-Startpunkte und/oder Pseudo-Endpunkte für die unregelmäßigen Herzzyklen bestimmt werden, die jeweils von längerer Dauer sind als regemäßige Zyklen, und dass basierend auf diesen Pseudo-Startpunkten und/oder Pseudo-Endpunkten Wellenformparameter/-kennlinien des unregelmäßigen Herzzyklus direkt gemessen oder berechnet werden; und
   dass unter Verwendung des modifizierten Wellenformdatensatzes ein Herz-Kreislaufparameter berechnet wird, wobei es sich bei dem Herz-Kreislaufparameter um einen Parameter handelt, der Vorlastabhängigkeit und Fluidreagibilität widerspiegelt, wie etwa Schlagvolumenschwankung, Pulsdruckschwankung, Systolendruckschwankung, Schlagvolumen, Herzzeitvolumen, systemische Gefäßnachgiebigkeit, Herzdurchfluss, Herzdurchflussgeschwindigkeit, Gefäßnachgiebigkeit oder vaskuläre Elastanz.

2. Computerimplementiertes Verfahren nach Anspruch 1, wobei das Bestimmen, ob es sich bei den einzelnen Herzzyklen um regelmäßige Herzzyklen oder um unregelmäßige Herzzyklen handelt, umfasst:

   dass eine oder mehrere Wellenformkennlinien des einzelnen Herzzyklus mit einer oder mehreren Wellenformkennlinien eines Kontroll-Herzzyklus verglichen werden; und
   dass der einzelne Herzzyklus als ein unregelmäßiger Herzzyklus identifiziert wird, wenn sich eine oder mehrere Wellenformkennlinien des einzelnen Herzzyklus um einen vorbestimmten Schwellenbetrag von der einen oder den mehreren Wellenformkennlinien des Kontroll-Herzzyklus unterscheiden.

3. Computerimplementiertes Verfahren nach Anspruch 1, wobei das Bestimmen des Pseudo-Endpunkts für einen unregelmäßigen Zyklus umfasst:

   dass ein Zeitfaktor für alle regelmäßigen Herzzyklen bestimmt wird;
   dass basierend auf den Diastolendrücken der Zyklen ein Druckfaktor bestimmt wird;
   dass ein Startpunkt des unregelmäßigen Zyklus identifiziert wird und Druckdatenpunkte identifiziert werden, die innerhalb eines auf den Startpunkt bezogenen, vorab festgelegten Bereichs gelegen sind;
   dass, wenn ein Druckdatenpunkt innerhalb des vorab festgelegten Bereichs gelegen ist und die Beziehung zwischen dem Druckdatenpunkt und dem Druckfaktor innerhalb eines vorbestimmten Bereichs liegt, dieser Druckdatenpunkt als Pseudo-Endpunkt zugewiesen wird; und
   dass, wenn mehr als ein Druckdatenpunkt innerhalb des Bereichs gelegen sind und die Beziehung zwischen einem jeden aus der Mehrzahl von Druckdatenpunkten und dem Druckfaktor innerhalb eines vorbestimmten Bereichs liegt, jener Druckdatenpunkt mit einem Zeitwert, der am nächsten bei dem Zeitfaktor gelegen ist, als Pseudo-Endpunkt zugewiesen wird.

4. Computerimplementiertes Verfahren nach Anspruch 3, wobei der auf den Startpunkt bezogene, vorab festgelegte Bereich von dem Startpunkt plus (einen Bereichsstartfaktor * die mittlere Zeitdauer) bis zu dem Startpunkt plus (einen Bereichsstoppfaktor * der mittleren Zeitdauer) reicht.

5. Computerimplementiertes Verfahren nach Anspruch 4, wobei der Bereichsstartfaktor 0,9 beträgt und der Bereichsstoppfaktor 1,1 beträgt.

6. Computerimplementiertes Verfahren nach Anspruch 1, wobei das Bestimmen des Pseudo-Startpunkts für einen unregelmäßigen Zyklus umfasst:

dass ein Zeitfaktor für alle regelmäßigen Herzzyklen bestimmt wird;
dass basierend auf den Diastolendrücken der Zyklen ein Druckfaktor bestimmt wird;
dass ein Endpunkt des unregelmäßigen Zyklus identifiziert wird und Druckdatenpunkte für die Wellenform identifiziert werden, die innerhalb eines auf den Endpunkt bezogenen, vorab festgelegten Bereichs gelegen sind;
dass, wenn ein Druckdatenpunkt innerhalb des vorab festgelegten Bereichs gelegen ist und die Beziehung zwischen dem Druckdatenpunkt und dem Druckfaktor innerhalb eines vorbestimmten Bereichs liegt, dieser Druckdatenpunkt als Pseudo-Startpunkt zugewiesen wird; und
dass, wenn mehr als ein Druckdatenpunkt innerhalb des Bereichs gelegen sind und die Beziehung zwischen einem jeden aus der Mehrzahl von Druckdatenpunkten und dem Druckfaktor innerhalb eines vorbestimmten Bereichs liegt, jener Druckdatenpunkt mit einem Zeitwert, der am nächsten bei dem Zeitfaktor gelegen ist, als Pseudo-Endpunkt zugewiesen wird.

7. Computerimplementiertes Verfahren nach Anspruch 6, wobei der auf den Startpunkt bezogene, vorab festgelegte Bereich von dem Startpunkt plus (einen Bereichsanfangsfaktor * die mittlere Zeitdauer) bis zu dem Startpunkt plus (einen Bereichsendfaktor * der mittleren Zeitdauer) reicht.

8. Computerimplementiertes Verfahren nach Anspruch 7, wobei der Bereichsanfangsfaktor 1,1 beträgt und der Bereichsendfaktor 0,9 beträgt.

9. Computerimplementiertes Verfahren nach Anspruch 1, wobei die Wellenformkennlinie die Standardabweichung eines unregelmäßigen Zyklus darstellt.

10. Computerimplementiertes Verfahren nach Anspruch 9, wobei die Standardabweichung ($\sigma_{irregular\_cycle}$) eines unre-gelmäßigen Zyklus dadurch berechnet wird, dass:

der Systolendruck ($P_{sys(nearest\_regular\_cycle)}$) des nächstgelegenen regelmäßigen Zyklus bestimmt wird;
die Standardabweichung ($\sigma_{nearest\_regular\_cycle}$) des nächstgelegenen regelmäßigen Zyklus bestimmt wird;
basierend auf dem respiratorischen Parameter und auf dem Systolendruck ($P_{sys(nearest\_regular\_cycle)}$) des nächstgelegenen regelmäßigen Zyklus der Systolendruck ($P_{sys(irregular\_cycle)}$) des unregelmäßigen Zyklus bestimmt wird; und
die folgende Gleichung gelöst wird:

$$\sigma_{irregular\_cycle} = \left( \frac{P_{sys(irregular\_cycle)}}{P_{sys(nearest\_regular\_cycle)}} \right) \times \sigma_{nearest\_regular\_cycle}$$

11. Computerimplementiertes Verfahren nach Anspruch 1, ferner umfassend, dass der Wellenformdatensatz mit einem Tiefpassfilter gefiltert wird.

12. Computerimplementiertes Verfahren nach Anspruch 1, ferner umfassend, dass die Position der geschätzten Herzzyklen auf einer grafischen Benutzerschnittstelle angezeigt wird.

13. Computerimplementiertes Verfahren nach Anspruch 1, ferner umfassend, dass, wenn ein unregelmäßiger Herzzyklus detektiert wird, auf einer grafischen Benutzerschnittstelle angezeigt wird, dass die Wellenform geschätzte Herzzyklen enthält.

14. Computerimplementiertes Verfahren nach Anspruch 1, wobei der Wellenformdatensatz aus einem Abtastzeitraum von festgesetzter Dauer stammt.

**15.** Computerlesbares Medium mit darauf gespeicherten, computerausführbaren Befehlen, welche, wenn sie in ein Verarbeitungssystem geladen und von diesem ausgeführt werden, das Verarbeitungssystem veranlassen, das Verfahren aus einem beliebigen der vorhergehenden Ansprüche durchzuführen.

## Revendications

**1.** Procédé mis en œuvre par ordinateur destiné à déterminer un paramètre cardiovasculaire, lequel comprend les étapes suivantes :

la réception d'un jeu de données de forme d'onde qui correspond à une pression artérielle donnée ou d'un signal qui est proportionnel au signal de la pression artérielle ou qui est dérivé de celui-ci ;
l'identification de cycles cardiaques individuels dans le jeu de données de forme d'onde ;
la mesure de caractéristiques de forme d'onde pour les cycles cardiaques individuels ;
la détermination des cycles cardiaques individuels en tant que cycles cardiaques réguliers ou cycles cardiaques irréguliers ;
**caractérisé par**
la mesure d'un paramètre respiratoire qui représente une variation d'origine respiratoire dans le jeu de données de forme d'onde, laquelle est causée par la respiration ;
la création d'un jeu de données de forme d'onde modifié qui contient les caractéristiques de forme d'onde des cycles cardiaques réguliers et les caractéristiques de forme d'onde des cycles cardiaques irréguliers, les caractéristiques de forme d'onde des cycles cardiaques irréguliers étant remplacées par des caractéristiques de forme d'onde estimées, ledit remplacement des caractéristiques de forme d'onde des cycles cardiaques irréguliers par des caractéristiques de forme d'onde estimées comprenant :

la détermination de pseudo-points de départ et/ou de pseudo-points finaux pour les cycles cardiaques irréguliers respectivement plus longs que les cycles réguliers, et ce sur la base des caractéristiques de forme d'onde de cycles cardiaques réguliers, et la mesure directe ou le calcul de paramètres/caractéristiques de forme d'onde du cycle cardiaque irrégulier sur la base desdits pseudo-points de départ et/ou pseudo-points finaux ; et
le calcul d'un paramètre cardiovasculaire en utilisant le jeu de données de forme d'onde modifié, le paramètre cardiovasculaire étant un paramètre qui reflète la précharge-dépendance et la réactivité au fluide, tel que la variation du volume systolique, la variation de la pression pulsée, la variation de la pression systolique, le volume d'éjection systolique, le débit cardiaque, la compliance vasculaire systémique, le flux cardiaque, la vitesse du flux cardiaque, la compliance vasculaire ou l'élastance vasculaire.

**2.** Procédé mis en oeuvre par ordinateur selon la revendication 1, lors duquel la détermination des cycles cardiaques individuels en tant que cycles cardiaques réguliers ou cycles cardiaques irréguliers comprend :

la comparaison d'une ou de plusieurs caractéristiques de forme d'onde du cycle cardiaque individuel avec une ou plusieurs caractéristiques de forme d'onde d'un cycle cardiaque de contrôle ; et
l'identification du cycle cardiaque individuel en tant que cycle cardiaque irrégulier lorsqu'une ou plusieurs caractéristiques de forme d'onde du cycle cardiaque individuel diffère(nt) d'un montant seuil prédéfini de ladite une caractéristique ou desdites plusieurs caractéristiques de forme d'onde du cycle cardiaque de contrôle.

**3.** Procédé mis en oeuvre par ordinateur selon la revendication 1, lors duquel la détermination du pseudo-point final pour un cycle irrégulier comprend :

la détermination d'un facteur de temps pour tous les cycles cardiaques réguliers ;
la détermination d'un facteur de pression sur la base des pressions diastoliques des cycles ;
l'identification d'un point de départ du cycle irrégulier et l'identification de points de données de pression qui se situent à l'intérieur d'une zone préétablie se référant au point de départ ;
lorsqu'un point de données de pression se situe à l'intérieur de la zone préétablie et que la relation entre le point de données de pression et le facteur de pression se situe à l'intérieur d'une plage prédéterminée, ledit point de données de pression est désigné comme pseudo-point final ; et
lorsque plus d'un point de données de pression se situe à l'intérieur de ladite zone et que la relation entre chaque point de la pluralité des points de données de pression et le facteur de pression se situe à l'intérieur d'une plage prédéterminée, le point de données de pression ayant une valeur de temps qui se situe le plus près du facteur de temps est désigné comme pseudo-point final.

4. Procédé mis en oeuvre par ordinateur selon la revendication 3, lors duquel la zone préétablie se référant au point de départ s'étend du point de départ plus (un facteur de départ de zone * la durée de temps moyenne) jusqu'au point de départ plus (un facteur d'arrêt de zone * la durée de temps moyenne).

5. Procédé mis en oeuvre par ordinateur selon la revendication 4, lors duquel le facteur de départ de zone est de 0,9 et le facteur d'arrêt de zone est de 1,1.

6. Procédé mis en oeuvre par ordinateur selon la revendication 1, lors duquel la détermination du pseudo-point de départ pour un cycle irrégulier comprend :

la détermination d'un facteur de temps pour tous les cycles cardiaques réguliers ;
la détermination d'un facteur de pression sur la base des pressions diastoliques des cycles ;
l'identification d'un point final du cycle irrégulier et l'identification de points de données de pression pour la forme d'onde lesquels se situent à l'intérieur d'une zone préétablie se référant au point final ;
lorsqu'un point de données de pression se situe à l'intérieur de la zone préétablie et que la relation entre le point de données de pression et le facteur de pression se situe à l'intérieur d'une plage prédéterminée, ledit point de données de pression est désigné comme pseudo-point de départ ; et
lorsque plus d'un point de données de pression se situe à l'intérieur de ladite zone et que la relation entre chaque point de la pluralité des points de données de pression et le facteur de pression se situe à l'intérieur d'une plage prédéterminée, le point de données de pression ayant une valeur de temps qui se situe le plus près du facteur de temps est désigné comme pseudo-point final.

7. Procédé mis en oeuvre par ordinateur selon la revendication 6, lors duquel la zone préétablie se référant au point de départ s'étend du point de départ plus (un facteur de début de zone * la durée de temps moyenne) jusqu'au point de départ plus (un facteur de fin de zone * la durée de temps moyenne).

8. Procédé mis en œuvre par ordinateur selon la revendication 7, lors duquel le facteur de début de zone est de 1,1 et le facteur de fin de zone est de 0,9.

9. Procédé mis en oeuvre par ordinateur selon la revendication 1, lors duquel la caractéristique de forme d'onde représente l'écart type d'un cycle irrégulier.

10. Procédé mis en oeuvre par ordinateur selon la revendication 9, lors duquel l'écart type ($\sigma_{\text{irregular\_cycle}}$) d'un cycle irrégulier est calculé :

en déterminant la pression systolique ($P_{\text{sys(nearest\_regular\_cycle)}}$) du cycle régulier le plus proche ;
en déterminant l'écart type ($\sigma_{\text{nearest\_regular\_cycle}}$) du cycle régulier le plus proche ;
en déterminant la pression systolique ($P_{\text{sys(irregular\_cycle)}}$) du cycle irrégulier sur la base du paramètre respiratoire et de la pression systolique ($P_{\text{sys(nearest\_regular\_cycle)}}$) du cycle régulier le plus proche ; et
en résolvant l'équation suivante :

$$\sigma_{\text{irregular\_cycle}} = \left( \frac{P_{\text{sys(irregular\_cycle)}}}{P_{\text{sys(nearest\_regular\_cycle)}}} \right) \times \sigma_{\text{nearest\_regular\_cycle}}$$

11. Procédé mis en oeuvre par ordinateur selon la revendication 1, lequel comprend en outre le filtrage du jeu de données de forme d'onde grâce à un filtre passe-bas.

12. Procédé mis en oeuvre par ordinateur selon la revendication 1, lequel comprend en outre l'affichage de la position des cycles cardiaques estimés sur une interface utilisateur graphique.

13. Procédé mis en oeuvre par ordinateur selon la revendication 1, lequel comprend en outre, lorsqu'un cycle cardiaque irrégulier est détecté, l'affichage sur une interface utilisateur graphique que la forme d'onde contient des cycles cardiaques estimés.

14. Procédé mis en œuvre par ordinateur selon la revendication 1, lors duquel le jeu de données de forme d'onde provient d'une période d'échantillonnage de durée déterminée.

15. Support lisible par ordinateur sur lequel sont stockées des instructions exécutables par ordinateur qui, lorsqu'elles sont chargées dans un système de traitement et sont exécutées par celui-ci, font en sorte que ledit système de traitement mette en oeuvre le procédé selon l'une quelconque des revendications précédentes.

FIG. 1

FIG. 2

FIG. 3

FIG. 4

FIG. 5

FIG. 6

FIG. 7

FIG. 8

900

910

**FIG. 9**

1000

1020   1010

**FIG. 10**

FIG. 11

FIG. 12

1300

1320

1310

## FIG. 13

1400

1410

1420

## FIG. 14

FIG. 15

FIG. 16

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4236527 A, Newbower
- US 4507974 A, Yelderman
- US 5146414 A, McKown
- US 5687733 A, McKown
- US 5400793 A, Wesseling
- US 5535753 A, Petrucelli
- US 7422562 B
- US 2003109791 A1
- US 20050187481 A1
- US 2009048527 A1
- WO 61151670 A
- US 20050187481 A